(19) Europäisches Patentamt
European Patent Office
Office européen des brevets

(11) **EP 0 719 560 B1**

(12) **FASCICULE DE BREVET EUROPEEN**

(45) Date de publication et mention
de la délivrance du brevet:
**02.07.2003 Bulletin 2003/27**

(51) Int Cl.7: **A61K 39/10**, C07K 14/235,
C12N 1/20, C07K 16/12

(21) Numéro de dépôt: **95402838.7**

(22) Date de dépôt: **15.12.1995**

(54) **Vaccin de type acellulaire antibordetella**

Antibordetella azellulärer Impfstoff

Antibordetella acellular vaccine

(84) Etats contractants désignés:
**AT BE CH DE DK ES FR GB IE IT LI NL SE**

(30) Priorité: **15.12.1994 FR 9415137**

(43) Date de publication de la demande:
**03.07.1996 Bulletin 1996/27**

(60) Demande divisionnaire:
**03010630.6**

(73) Titulaire: **INSTITUT PASTEUR**
**75724 Paris Cédex 15 (FR)**

(72) Inventeurs:
• **Gueirard, Pascale**
**F-92360 Meudon la Foret (FR)**
• **Guiso, Nicole**
**F-75013 Paris (FR)**

(74) Mandataire: **Desaix, Anne et al**
**Ernest Gutmann - Yves Plasseraud S.A.**
**3, rue Chauveau-Lagarde**
**75008 Paris (FR)**

(56) Documents cités:
**EP-A- 0 787 796        WO-A-90/13312**

• INFECTION AND IMMUNITY, vol. 61, no. 10, pages 4072-4078, XP002001710 GUEIRARD ET AL: "VIRULENCE OF BORDETELLA BRONCHISEPTICA:ROLE OF ADENYLATE CYCLASE-HEMOLYSIN"
• INFECTION AND IMMUNITY, vol. 60, no. 2, pages 571-577, XP002001711 BEATTIE ET AL: "A VIR-REPRESSED GENE OF BORDETELLA PERTUSSIS IS REQUIRED FOR VIRULENCE"
• JOURNAL OF BACTERIOLOGY, vol. 170, no. 11, pages 5059-5066, XP002001712 KNAPP ET AL: "TWO TRANS-ACTING REGULATORY GENES (VIR AND MOD) CONTROL ANTIGENIC MODULATION IN BORDETELLA PERTUSSIS"
• DATABASE MEDLINE FILE SERVER STN KARLSRUHE ABR G 93123171, BEATTIE ET AL: "REPRESSOR BINDING TO A REGULATORY SITE IN THE DNA CODING SEQUENCE IS SUFFICIENT TO CONFER TRANSCRIPTIONAL REGULATION OF THE VIR-REPRESSED GENES (VRG GENES) IN BORDETELLA PERTUSSIS" XP002001714
• CHEM. ZENTRALBLATT NR.8-1284,1966 PAGE 2576,ABR G 1292 & KITASATO ARCH.EXP.MED.35,1-11,1962 XP002001713
• BOURSAUX-EUDE C. ET AL: 'Intranasal murine model of Bordetella pertussis infection: II. Sequence variation and protection induced by a tricomponent acellular vaccine.' VACCINE vol. 17, 1999, pages 2651 - 2660
• DATABASE BIOSIS BREV198682000368, 1986 MUSSER J.M. ET AL: 'GENETIC DIVERSITY AND RELATIONSHIPS IN POPULATIONS OF BORDETELLA-SPP'
• DATABASE BIOSIS PREV199800495628, 1998 YUK M.H. ET AL: 'Human but not ovine isolates of Bordetella parapertussis are highly clonal as determined by PCR-based RAPD fingerprinting.'

Remarques:
Le dossier contient des informations techniques présentées postérieurement au dépôt de la demande et ne figurant pas dans le présent fascicule.

EP 0 719 560 B1

**Description**

Le genre <u>Bordetella</u> comprend quatre espèces <u>Bordetella pertussis</u>, <u>Bordetella parapertussis</u>, <u>Bordetella bronchiseptica</u> et <u>Bordetella avium.</u>

Les <u>Bordetellae</u> sont des coccobacilles gram-négatif responsables d'infections respiratoires. <u>Bordetella pertussis</u> et <u>Bordetella parapertussis</u>, agents de la coqueluche, sont des pathogènes strictement humains. <u>Bordetella bronchiseptica</u> est pathogène pour divers mammifères et, plus rarement pour l'homme et à la différence de <u>B. pertussis</u> et <u>B. parapertussis</u> est capable de survivre à l'extérieur de l'hôte. Bordetella avium n'est pathogène que pour l'oiseau.

Depuis l'introduction de la vaccination coquelucheuse dans les pays où-la couverture vaccinale est supérieure à 80%, on a pu observer une chute spectaculaire de la morbidité et de la mortalité. Cette chute est bien attribuable à la vaccination puisque dans plusieurs pays (Grande-Bretagne, Suède, Japon...) des épidémies meurtrières de coqueluche ont eu lieu dans les années qui ont suivi l'arrêt de la vaccination.

L'invention propose des compositions immunogènes pouvant entrer dans la constitution de vaccins coquelucheux, ces compositions étant de type "acellulaire" et présentant une efficacité au moins identique à celle du vaccin connu.

L'invention concerne d'une part des vaccins utilisables en médecine vétérinaire et d'autre part des vaccins utilisables en médecine humaine.

Le vaccin coquelucheux utilisé actuellement est un vaccin cellulaire, composé de suspensions bactériennes de <u>B. pertussis</u> (mélange de deux souches différant dans l'expression d'agglutinogènes) inactivées par chauffage. Ce vaccin est généralement utilisé sous forme combinée avec des fractions purifiées diphtérique et tétanique, le composant haemophilus et le composant viral polio inactivé. La vaccination consiste en trois injections à un mois d'intervalle dès l'age de deux mois et une injection à 18 mois. Aucune autre injection de rappel n'est ensuite pratiquée.

Ce vaccin est parfois mal toléré, tant localement que généralement. Il a, en particulier, été accusé d'entraîner des complications neurologiques graves à type d'encéphalite aigüe ; cependant, des études très récentes semblent conclure à l'absence de preuve statistique d'une relation entre le vaccin cellulaire et les complications neurologiques sévères (Griffiths AH. Vaccine 1989 ; 7:199-210).

Il n'en demeure pas moins vrai que le vaccin cellulaire est mal toléré et est responsable d'effets, réversibles mais indésirables. Pour ces raisons, un nouveau vaccin dépourvu de ces effets est souhaitable. Pour envisager la définition d'un nouveau vaccin, il apparaissait nécessaire de caractériser certains facteurs impliqués dans la virulence de la bactérie et le cas échéant dans la régulation de la virulence. Purifiés, ces différents facteurs sont autant de candidats théoriques pour la constitution d'un vaccin coquelucheux dit "acellulaire" par opposition au vaccin classique. Ce nouveau type de vaccin devrait apporter, en plus d'une meilleure tolérance, une efficacité au moins égale à celle du vaccin classique.

Les facteurs impliqués dans la virulence de <u>B. pertussis</u> ont été identifiés comme suit : la coqueluche peut être schématiquement définie par l'association d'un syndrome infectieux, impliquant l'adhésion des bactéries aux cellules cibles (cellules ciliées de l'appareil respiratoire), sans invasion ni dissémination dans l'organisme de l'hôte et d'un syndrome toxique secondaire incluant des effets cytopathogènes locaux, électifs pour l'épithélium respiratoire cilié (destruction et élimination des cellules ciliées, accumulation du mucus, réaction inflammatoire) et des effets systémiques dont le plus évident est l'hyperleucocytose avec hyperlymphocytose.

Grâce aux techniques récentes de biologie moléculaire, un certain nombre de facteurs impliqués dans la virulence de <u>B. pertussis</u> ont été caractérisés et la régulation de leur expression connue. Ces facteurs peuvent être classés en deux catégories, ceux participant au syndrome infectieux (adhésines) et ceux jouant un rôle dans le syndrome toxinique (toxines).

**Les adhésines:**

- l'hémagglutinine filamenteuse ou **FHA** est considérée comme jouant un rôle majeur dans l'adhésion de la bactérie sur l'épithélium cilié (Locht C., Bertin P., Menozzi F. D. and Renaud G. Mol. Microbiol. 1993, 9:653-66). La FHA est toujours exprimée par les souches virulentes et est sécrétée. Son gène de structure a été cloné et séquencé (Relman D. et al, 1989, Proc. Natl. Acad. Sci. USA, 86:2637-2641). Il code pour une protéine de 360 kDa, mais seul un fragment de 220 kDa peut être purifié. Cette protéine se fixe aux glycoprotéines des cellules ciliées et elle possède des sites de fixation pour les intégrines des lymphocytes et des macrophages. Il vient d'être montré récemment que la FHA présente une homologie avec certaines protéines des cellules endothéliales de l'hôte (Tuomanen E., Prasad S. M., George J. S., Hoepelman A. I. M., Ibsen P., Heron I., and Starzyk R. M. 1993. Proc. Natl. Sci. USA. 90:7824-7828).

- Les deux agglutinogènes ou **AGG** de <u>B. pertussis</u> permettent de classer les souches en sérotypes. Deux AGG ont été caractérisés. Ces protéines sont sécrétées et jouent un rôle dans l'adhésion de la bactérie aux cellules épithéliales (Mooi F. Van der Heide H. G. D., Ter Avest A. R., Welinder K.G., Livey I., Van der Zeijst B.A.M., and Gaastra, W. 1987. Microb. Pathog. 2:473-484).

- La pertactine ou **PRN**, est une protéine de 93 kDa, mais seul un fragment de 69 kDa peut être purifié. Cette protéine possède deux sites de fixation pour les intégrines des macrophages et des lymphocytes (Charles I, Dougan G., Pickard D., Chattfield S. Smith M. Novotny P., Morissey P. and Fairweather N.F. 1989 Proc Natl Acad Sci. 86: 3554-3558).

- La toxine de pertussis ou **PTX**, toxine de type A-B, sécrétée qui, outre ses effets cytopathogènes, participe à l'adhésion par l'intermédiaire de sa sous-unité B. L'oligomère B est capable de se fixer sur les récepteurs des cellules ciliées mais pas nécessairement sur les mêmes récepteurs que ceux de la FHA. La fixation de la PTX sur les leucocytes empêcherait leur migration vers le site de la réaction inflammatoire. Cette fixation induirait une augmentation du nombre de molécules d'intégrines fonctionnelles sur les leucocytes ce qui favoriserait la fixation de la bactérie par l'intermédiaire de la FHA (Rozindski E., Burnette W.N., Jones T., Mar V., and Tuomanen E. 1993 J. Exp. Med. 178:917-924).

**Les toxines:**

- La toxine de pertussis ou **PTX** est sécrétée et considérée comme la toxine majeure de B. pertussis. Sa **sous-unité A,** possède une activité ADP-ribosyl transférase. Après fixation de la partie B de la toxine sur la cellule cible, cette sous-unité A est capable de pénétrer dans la cellule, d'inactiver les protéines régulatrices G et d'entraîner ainsi une perturbation de toutes les fonctions cellulaires. C'est ce facteur qui serait responsable des effets biologiques généralisés observés lors de la maladie tels l'hyperlymphocytose, l'hyperinsulinémie, la sensibilité à l'histamine.

- La toxine dermonécrotique ou **TDN**, non encore bien caractérisée et la toxine cytotrachéale ou **TCT,** petite glycoprotéine sécrétée de la famille des muramyl peptides, dérivée du peptidoglycane de la bactérie, agiraient de concert pour détruire les cellules ciliées de l'appareil respiratoire de l'hôte. La TCT empêche de plus la régénération de l'épithélium respiratoire (Luker K., Collier J. L., Kolodziej E. W., Marshall G.R., and Goldman W.E. 1993. Proc. Natl. Acad. Sci. USA. 90:2365-2369).

- L'adényl cyclase-hémolysine ou **AC-Hly**, est une protéine bifonctionnelle, possédant une activité adényl cyclase et une activité hémolytique. Elle est sécrétée par la bactérie. Son gène de structure a été cloné et séquencé (Glaser P. et al, 1988, Molec. Microb. 2, 19-20). Il s'avère que cette protéine fait partie de la famille des toxines dites "RTX" pour "repeats in toxins" et présente des homologies avec l'hémolysine d'Escherichia coli, d'Actinobacillus pleuropneumoniae, les leucotoxines de Pasteurella haemolytica et d'Actinobacillus actinomycetemcomitans. Cette protéine, comme la PTX, est capable de pénétrer dans les cellules eucaryotes telles que les macrophages, d'être activée par la calmoduline, de synthétiser de grandes quantités d'AMPc et de perturber les fonctions cellulaires (Coote J. 1992. FEMS Microbiol. Rev. 88:137-162).

De même, les facteurs impliqués dans la virulence de B. parapertussis et B. bronchiseptica ont été identifiés.
Gueirard et al. ont montré que l'adényl cyclase-hémolysine de B. bronchiseptica joue un rôle important dans la virulence de cette bactérie et constitue un antigène protecteur important contre les infections par B. bronchiseptica (Inf. Immun. 1993, vol. 61, n° 10, p. 4072-4078).
Les infections à B. pertussis, B. parapertussis et B. bronchiseptica sont indistinguables d'un point de vue clinique. Ces bactéries ont plus de 75% d'homologie au niveau de l'ADN. Elles n'ont été classifiées en espèces que sur la base de caractères phénotypiques et biochimiques. B. parapertussis et B. bronchiseptica synthétisent des facteurs de virulence très proches fonctionnellement et immunologiquement de B. pertussis à l'exception de la PTX.
Un vaccin composé de suspensions bactériennes de B. pertussis inactivées protège contre une infection à B. pertussis mais aussi contre une infection à B. parapertussis et à B. bronchiseptica dans le modèle murin. Bien qu'il n'y ait pas de données épidémiologiques sur les infections à B. parapertussis en France, il est à noter que l'on isole peu de souches de cette espèce dans notre pays, pays vacciné depuis 25 ans avec un "vaccin pertussis", alors qu'elles sont isolées dans les pays non ou mal vaccinés.
Outre la présence de ces différentes adhésines et toxines, les Bordetelles sont caractérisées par une régulation de l'expression des facteurs impliqués dans leur virulence. Autrement dit, les Bordetelles subissent des variations et modulations de phase.
Les Bordetelles, suivant leur environnement, peuvent devenir "avirulentes" c'est à dire incapables d'induire la létalité, une réaction inflammatoire et des lésions pulmonaires dans le modèle murin d'infection respiratoire. Elles subissent soit une modulation de phase soit une variation de phase. La variation de phase s'observe à une fréquence

allant de $10^{-3}$ à $10^{-6}$ et est quasiment irréversible. Elle se traduit par un arrêt de l'expression des toxines et adhésines décrites précédemment et par l'expression d'autres facteurs non encore bien caractérisés (changement des bactéries "virulentes" Phase I en bactéries "avirulentes" Phase IV). Les bactéries de phase I et de phase IV ont été décrites par Lacey B. 1960, J. Hyg, 58:57-93. La modulation de phase, phénotypiquement semblable à la variation de phase est totalement réversible et se traduit par un arrêt de la synthèse des adhésines et des toxines lors de changements environnementaux (composition du milieu de culture, température...).

Knapp et al. ont déterminé deux séries de gènes dont l'expression pendant la phase de virulence est soit activée (vir activated genes, ou vag), soit réprimée (vir repressed gènes, ou vrg) (J. Bacteriol. 1988, vol. 170, n° 11 : p. 5059-5066).

La variation de phase et la modulation de phase observées chez Bordetella sont sous le contrôle de deux gènes régulateurs, bvg A et bvg S (Arico B. et al, 1989, Proc. Natl. Acad. Sci USA, 86: 6671-6675).

Le gène bvg S code pour une protéine sensible aux conditions extérieures. Cette protéine module par phosphorylation l'activité de la protéine codée par le gène bvgA, qui est d'une part un activateur positif de la transcription des gènes codant pour les facteurs de virulence (gènes vag pour "vir activated genes") cités précédemment (Uhl M. A. And Miller J, 1994. PRoc. Natl. Acad. Sci USA 91:1163-1167), d'autre part un répresseur de la transcription de certains gènes (Beattie D.T. et al, J. of Bacteriology, Jan 93, p. 159-527). Les gènes dont l'expression est réprimée sont appelés gènes vrg pour "vir repressed genes" et sont encore mal caractérisés. Il a cependant été montré que le gène vrg 6 de B. pertussis code pour une protéine ayant un rôle dans la persistance de la bactérie chez l'hôte (Beatties D. et al, 1992, Infect. Imm. 60:571-577). Chez B. bronchiseptica, deux protéines codées par les gènes vrg ont été caractérisées : ce sont des protéines de type flagelles (B. bronchiseptica Phase I est une bactérie immobile ne synthétisant pas de flagelles mais synthétisant adhésines et toxines et B. bronchiseptica Phase IV est une bactérie mobile synthétisant des flagelles).

Afin de mesurer la virulence des bactéries et d'évaluer les effets toxiniques locaux et généraux, un modèle d'infection respiratoire de la souris a été mis au point (Guiso N. et al, 1991, Microb. Pathogen 11, 423-431). A l'aide de ce modèle murin, il a pu être montré que la , PTX inactivée chimiquement ou génétiquement, est un bon immunogène. Cette anatoxine a une activité protectrice contre des infections létales à B. pertussis, mais ne semble pas induire la synthèse d'anticorps efficaces contre la persistance de la bactérie. (Khelef. N. Danve B. Quentin-Millet M. J and Guiso N. 1993 Infect Immun. 64:486-490).

Ces résultats relatifs à la virulence des Bordetelles et à la régulation de cette virulence, montrent que la coqueluche est une maladie multifactorielle et que le vaccin doit non seulement protéger contre des infections létales mais aussi contre la persistance de la bactérie. Des conclusions similaires s'appliquent aux infections dues à B. parapertussis ou à B. bronchiseptica.

Des tentatives de développement d'un vaccin acellulaire, à partir des composants isolés de la famille des adhésines ou des toxines ont été effectuées. Ainsi, des compositions acellulaires contenant soit la toxine de B. pertussis (PTX) purifiée soit cette toxine associée à l'hémagglutinine filamenteuse (FHA) purifiée ont été préparées.

Les premiers essais de tolérance chez l'homme de ces compositions acellulaires (PTX ou PTX-FHA) montrent une nette diminution des complications tant locales (douleur, gonflement) que générales (fièvre, convulsions..) en comparaison avec le vaccins cellulaire classique (Edward K., J. Infect. Dis. 1993, 168, 15-20).

Ces nouvelles préparations (PTX ou PTX-FHA) ont une bonne immunogénicité et induisent un taux élevé d'anticorps. Cependant, la recherche d'anticorps vaccinaux est une méthode imparfaite car une séroconversion n'est pas synonyme de protection contre la maladie et aucune démonstration n'a été faite du caractère protecteur des anticorps obtenus, ni du taux éventuel de protection.

Les résultats d'essais cliniques de différents vaccins cellulaires et acellulaires ont été publiés (International Symposium on Pertussis Vaccine trials, Rome 30.10.95-1.11.95). Ces résultats montrent que tous les vaccins cellulaires ne sont pas équivalents, certains sont très efficaces et induisent peu d'effets secondaires et d'autres sont très peu efficaces et induisent des effets secondaires plus importants.

Les résultats publiés montrent que les vaccins acellulaires testés, monovalent (PTX), bivalent (PTX, FHA), trivalent (PTX, FHA, PRN) ou pentavalent (PTX, FHA, PRN, AGG2, AGG3) induisent très peu d'effets secondaires, sont tous immunogènes et ont tous une efficacité contre la maladie (selon la définition OMS) supérieure ou égale à 70%. Cependant, l'efficacité d'un vaccin acellulaire PTX-FHA est toujours inférieure quelle que soit la définition de cas, à celle d'un vaccin cellulaire efficace.

Malgré les résultats encourageants obtenus s'agissant de l'immunogénicité de différentes compositions contenant à la fois des adhésines et des toxines de B. pertussis, les inventeurs ont estimé qu'une protection efficace contre la maladie due à une infection par B. pertussis, B. parapertussis ou B. bronchiseptica, nécessitait de considérer des facteurs supplémentaires par rapport aux adhésines et toxines et en particulier des facteurs intervenant dans la persistance de la bactérie.

Leurs observations les ont conduits à définir un modèle décrit en détail dans la partie expérimentale, à partir duquel de nouveaux critères préalables à la définition de vaccins ont été définis.

Ainsi, selon la présente demande, pour définir des vaccins efficaces et substantiellement non toxiques, contre l'une au moins des Bordetelles B. pertussis, B. parapertussis ou B. bronchiseptica, il convient de mettre en oeuvre non seulement une ou plusieurs adhésines et/ou toxines de ces bactéries, mais également un ou plusieurs facteurs dont la synthèse est réprimée lorsqu'il y a expression des toxines et adhésines de la bactérie. Ces facteurs sont notamment des produits d'expression des gènes vrg dont il a été question précédemment.

Dans le but de préparer un vaccin coquelucheux dépourvu d'effets secondaires et protecteur contre les effets locaux ou systémiques dus aux toxines synthétisées par B. pertussis et/ou B. parapertussis et/ou B. bronchiseptica, et protecteur contre la persistance chez l'hôte de ces bactéries, l'invention a pour objet une composition immunogène, caractérisée en ce qu'elle comprend une protéine adénylcyclase-hémolysine (AC-Hly) ou une partie immunogène de cette AC-Hly, caractéristique d'une souche de Bordetelle choisie parti B. pertussis, B. parapertussis ou B. bronchiseptica et en ce qu'elle comprend en outre un extrait bactérien contenant les produits des gènes vrg d'une souche de Bordetelle choisie parmi B. pertussis, B. parapertussis ou B. bronchiseptica ou une partie de ces produits d'expression, suffisante pour induire une réponse immunitaire chez un hôte auquel l'extrait serait administré.

La demande de brevet internationale WO 90/13312 décrit l'utilisation d'une préparation vaccinante élaborée à partir d'une adényl cyclase purifiée d'une bactérie donnée du genre Bordetella, pour la préparation d'un antigène protecteur pour l'homme ou l'animal contre les infections et les effets toxiques causés par une bactérie du genre Bordetella.

Selon les inventeurs, cette protéine devrait être incorporée dans une composition vaccinante dès lors qu'elle joue un rôle important dans la virulence des Bordetelles pour les raisons suivantes :

*   elle est exprimée très précocément après l'infection puisque des anticorps spécifiques sont synthétisés et détectés, aussi bien chez l'homme infecté que chez l'animal infecté, dès le début de l'infection (Khelef N, Sakamoto H., and Guiso N 1992. Microbiol. Pathogen. 12:227-235 et Gueirad P. and Guiso N. 1993 Infect. Immun. 61:4072-4078).

*   elle est nécessaire à la bactérie pour initier l'infection (Khelef N. Sakamoto H. and Guiso N. 1992 Microbiol. Pathogen. 12:227-235).

*   son utilisation sous forme purifiée comme antigène dans le modèle murin d'infection respiratoire protège les souris contre la colonisation de l'appareil respiratoire (Guiso , Szatanik, et Rocancourt . 1989 Microb. Pathogen. 11: 423-431).

*   elle est responsable, in vitro, de la mort des macrophages alvéolaires par apoptose (Khelef, Zyglinski, et Guiso Infect. Immun. 61:4064-4071). Les autres adhésines ou toxines n'interviennent pas dans le processus d'apoptose.

Par ailleurs, la présence dans la composition immunogène, d'un extrait bactérien comprenant les produits d'expression des gènes vrg permettrait d'améliorer la réponse immunitaire humorale et/ou cellulaire obtenue après infection chez les sujets vaccinés et contribuerait également à la protection contre la persistance de la bactérie.

L'extrait bactérien dit "extrait bactérien vrg" dont question ci-dessus contient tous les constituants de la membrane externe, y compris l'endotoxine LPS, d'une bactérie de phase IV, c'est à dire d'une bactérie n'exprimant pas les gènes vag. L'endotoxine LPS peut alternativement être éliminée ou détoxifiée.

Cet extrait peut être présent sous forme de suspension.

Une première composition immunogène préférée de l'invention est une composition immunogène caractérisée en ce qu'elle comprend en outre une ou plusieurs adhésines ou toxines respectivement de B. pertussis, B. parapertussis ou B. bronchiseptica, choisie(s) parmi la FHA, les AGG ou la PRN et la PTX.

Les adhésines des Bordetella renforcent le caractère immunogène de la composition contenant la AC-Hly et l'extrait bactérien vrg.

Selon un premier mode de réalisation de l'invention, la composition immunogène est caractérisée en ce qu'elle comprend la toxine PTX de B. pertussis, la toxine AC-Hly de B. pertussis et les adhésines FHA et PRN et un extrait bactérien contenant les protéines codées par les gènes vrg de B. pertussis ou une partie de ces protéines, suffisante pour induire une réponse immunitaire chez un hôte auquel l'extrait serait administré.

Une telle composition peut être mise en oeuvre pour préparer des vaccins protecteurs contre les effets létaux et systémiques de B. pertussis chez l'homme et contre la persistance de la bactérie.

Selon un mode de réalisation préféré de l'invention, la toxine AC-Hly de B. pertussis est également contenue dans un extrait bactérien contenant tout ou partie des facteurs de virulence du groupe des adhésines ou des toxines de B. pertussis.

Les extaits bactériens "vag" ou "vrg" auxquels il est fait appel pour réaliser l'invention, sont de préférence des extraits dits "extraits urée". de

Un "extrait urée" est composé d'un mélange de protéines exprimées à la surface de la bactérie et qui sont séparées de la bactérie après incubation de celle-ci avec de l'urée 5M. L'"extrait urée vag" de B. bronchiseptica par exemple contient entre autres protéines l'AC-Hly, la FHA, la PRN et le LPS (lipopolysaccharide endotoxine) et l'"extrait urée vrg" contient, plusieurs protéines non encore caractérisées, les flagelles et le LPS.

L'utilisation d'extraits urée permet notamment la réalisation d'un vaccin de moindre coût par rapport à un vaccin qui serait obtenu à partir des protéines contenues dans les extraits, sous forme purifiée.

En outre, les inventeurs ont constaté que les extraits urée utilisés peuvent induire une réponse immunitaire cellulaire de type T (lymphoprolifération), se comportant ainsi comme le vaccin cellulaire utilisé jusqu'à ce jour.

Au contraire, les compositions exclusivement acellulaires n'induiraient pas de réponse T, réaction qui se produit pourtant en cas d'infection.

Les extraits urée vag ou vrg sont préparés respectivement à partir des bactéries de phase I ou de phase IV. Le cas échéant, les bactéries de phase IV sont remplacées par des bactéries dont le gène bvgS est muté de façon telle que les bactéries n'expriment que les protéines codées par les gènes vrg.

La préparation de ces extraits est décrite en détail dans la partie expérimentale.

Ainsi, l'invention concerne de façon préférée, une composition immunogène comprenant à la fois un extrait urée vag de B. pertussis et un extrait urée vrg de B. pertussis.

Une souche de B. pertussis appropriée pour la préparation de ces extraits est la souche HAV entrant dans le cadre de l'invention et déposée à la CNCM (Collection Nationale de Cultures de Microorganismes à Paris), le 19 Octobre 1994 sous le n° I-1485. Pour préparer l'extrait urée vag, la souche HAV peut être utilisée directement puisqu'il s'agit d'une souche de phase I.

En revanche, l'extrait urée vrg est obtenu à partir d'une souche de phase IV dérivée de la souche de phase I par exemple par mutation du gène bvgS de la bactérie ou par culture de ladite souche de phase I dans un milieu contenant uniquement du sulfate de magnésium, de façon à obtenir l'expression des seuls gènes vrg de B. pertussis.

L'invention propose en outre des compositions immunogènes élaborées selon les principes décrits ci-dessus pour B. pertussis, à partir des Bordetelles de la famille de B. parapertussis ou de B. bronchiseptica.

Il est en effet connu que la vaccination avec le vaccin cellulaire disponible sur le marché, c'est à dire composé de suspensions bactériennes de bactéries de phase I de B. pertussis inactivées protège contre des infections B. pertussis et à B. parapertussis dans le modèle murin. Cependant, il a été démontré récemment que l'administration des facteurs purifiés de B. pertussis (PTX ou FHA ou PRN) ne protège pas contre la maladie et l'infection dues à B. parapertussis. De même l'administration de l'AC-Hly purifiée de B. parapertussis ne protège pas contre une infection à B. pertussis dans le modèle murin.

Ces résultats suggèrent que malgré une homologie très élevée entre les deux espèces, la protection est spécifique d'espèce. L'utilisation de vaccins acellulaires, composés de PTX, PRN et FHA des seules souches B. pertussis, comme vaccins coquelucheux dans les années à venir, risquerait donc d'entraîner une augmentation des infections à B. parapertussis (Khelef Danve Quentin-Millet et Guiso 1993, Infect. Immun. 61:46-490 et Gueirad P., et Guiso 1993 Infect. Immun 61:4072-4078).

L'invention propose donc également des compositions immunogènes caractérisées en ce qu'elles comprennent la toxine AC-Hly de B. parapertussis et un extrait bactérien contenant tout ou partie des protéines codées par les gènes vrg de B. parapertussis.

Avantageusement, la toxine AC-Hly de B. parapertussis est contenue dans un extrait bactérien contenant tout ou partie des facteurs de virulence du groupe des adhésines ou des toxines de B. parapertussis. Un tel extrait est de préférence un extrait urée vag, obtenu par exemple à partir de B. parapertussis souche n° 1 déposée à la CNCM le 2 Décembre 1994, sous le n° I-1498. La souche de B. parapertussis n° I-1498 entre dans le cadre de la présente invention.

De même, l'extrait bactérien vrg est de préférence un extrait urée vrg obtenu par exemple à partir de B. parapertussis souche n° 1 déposée à la CNCM le 2 Décembre 1994, sous le n° I-1498, selon des modalités analogues à celles qui ont été décrites pour la souche B. pertussis.

Selon un autre aspect et en particulier dans le but de réaliser un vaccin vétérinaire, la présente demande propose des compositions immunogènes caractérisées en ce qu'elle comprennent la toxine AC-Hly de B. bronchiseptica et un extrait bactérien contenant tout ou partie des protéines codées par les gènes vrg de B. bronchiseptica.

Comme indiqué précédemment au sujet de B. pertussis et B. parapertussis, la toxine AC-Hly de B. bronchiseptica est contenue dans un extrait bactérien comprenant tout ou partie des facteurs de virulence (adhésines et/ou toxines) de B. bronchiseptica.

Il s'agit avantageusement d'un extrait urée vag, obtenu par exemple à partir de la souche B. bronchiseptica 973S déposée à la CNCM le 12 Mai 1989 sous le n° I-858.

De façon analogue, l'extrait urée vrg est de préférence obtenu à partir de la souche B. bronchiseptica 973S déposée à la CNCM le 12 Mai 1989 sous le n° I-858, selon les modalités décrites ci-dessus pour B. pertussis.

Selon un autre mode de réalisation particulier de l'invention, une composition immunogène capable d'induire la production d'anticorps contre B. bronchiseptica, telle que décrite ci-dessus, comprend des composants polypeptidiques caractéristiques des flagelles de B. bronchiseptica. Ces composants ont été décrits par Akerley B.J. et al (J. of Bact. Feb 1992, p. 980-990).

L'immunité humorale anti-Bordetelle a longtemps été considérée comme la seule importante. En effet, il est connu depuis très longtemps qu'après infection, on peut détecter des anticorps anti-PTX et anti-FHA dans le sérum des malades. De plus, il est possible de protéger passivement contre la maladie, dans le modèle murin, avec des anticorps anti-PTX. Les anticorps circulants jouent donc un rôle dans la neutralisation de la PTX et dans l'inhibition de l'attachement de la bactérie.

Cependant, il n'a jamais été mis en évidence de corrélation entre le taux d'anticorps spécifiques de ces antigènes dans le sérum des individus vaccinés ou infectés et la protection contre la maladie. Très peu de choses sont connues concernant l'immunité des autres facteurs, en particulier l'AC-Hly, la PRN et le LPS. Il a toutefois été montré

* qu'il est possible de protéger passivement des souris contre une infection à B. pertussis avec des anticorps anti-AC-Hly.
* que dans les sérums d'enfants infectés non vaccinés (dont l'âge est supérieur à 8 mois afin d'éviter la présence d'anticorps maternels qui peuvent fausser l'interprétation de la sérologie, et de moins de deux ans afin de connaître le passé clinique) ou des sérums de souris infectées avec des isolats cliniques de B. pertussis ou de B. bronchiseptica l'on peut détecter des anticorps anti-AC-Hly, très précocément, indiquant que cette protéine est exprimée in vivo et dès le début de l'infection, des anticorps anti-FHA, des anticorps anti-PTX, et des anticorps anti-LPS. Les anticorps anti-PRN apparaissent beaucoup plus tardivement et ne persistent pas très longtemps (Guiso, Grimprel, Anjak et Bégué et 1993, Eur. J. Clin. Microbiol. Infect. Dis. 12:596-600).

La vaccination avec le vaccin cellulaire induit aussi la synthèse d'anticorps contre ces facteurs alors que les vaccins acellulaires testés jusqu'à ce jour n'induisent des anticorps que contre les facteurs qui les constituent.

Diverses études suggèrent en outre que l'immunité cellulaire serait aussi requise pour la protection contre l'infection. Plusieurs équipes ont montré que les Bordetelles peuvent pénétrer dans différents types cellulaires, cellules épithéliales, lymphocytes, moncytes, et y persister, in vitro. Par ailleurs, à la suite d'études épidémiologiques, il a été observé que B. bronchiseptica peut persister chez l'homme très longtemps. Enfin, alors qu'il n'est pas possible d'isoler de bactéries plus de 40 jours après l'infection au niveau pulmonaire chez la souris, il est toujours possible de détecter de l'ADN par PCR. L'ensemble de ces résultats suggèrent que les Bordetelles pourraient persister intracellulairement chez l'hôte à un moment donné de l'infection et que l'immunité cellulaire pourrait être requise pour contrôler un état intracellulaire qui permettrait ainsi aux bactéries d'échapper aux défenses immunitaires de l'hôte.

La démonstration de l'implication des cellules T au cours de l'infection a été obtenue récemment (Redhead, Watkins, Barnard et Mills 1993, Infect. Immun. 61:3190-3198). En effet, à dose sublétale, les souris normales éliminent les bactéries en 30-40 jours alors que les souris déficientes en cellules T (souris "nude") sont incapables d'éliminer les bactéries et développent une infection chronique. Le transfert de cellules T de souris convalescentes chez les souris "nude" rend celles-ci capables d'éliminer les bactéries. De plus, les cellules de la rate de souris convalescentes produisent des taux élevés d'IL-2, IFN-γ et TNF, mais pas d'IL-4 et d'IL-5. Ce profil est caractéristique des sous-populations des cellules T que sont les cellules de type Th1. L'immunisation des souris avec le vaccin entier induit aussi une réponse de type Th1 et une réponse anticorps modérée alors que l'immunisation avec un vaccin acellulaire composé de PTX, FHA et PRN induit une réponse de type Th2 et un très haut taux d'anticorps. S'il existe une forte corrélation entre des taux élevés d'IgG sériques spécifiques d'antigènes de B. pertussis et une élimination de la bactérie des poumons, des réponses cellulaires directes sont cependant nécessaires pour une élimination complète de la bactérie. L'élimination des bactéries moins rapide chez les souris immunisées avec des vaccins acellulaires qu'avec des vaccins entiers qui ont été observés serait donc due au fait que le vaccin acellulaire ne favoriserait pas l'induction de cellules Th1. Quelque soit le mécanisme d'action des Th1, il est donc maintenant clair que les cellules T jouent un rôle important non seulement indirect en stimulant la synthèse d'anticorps mais aussi direct dans l'immunité anti-B. pertussis via le recrutement, la stimulation et l'activation de cellules phagocytaires, telles que les macrophages et les polynucléaires neutrophiles.

Il a en outre été observé que les Bordetelles peuvent persister chez l'homme, en particulier B. bronchiseptica. Dans certains cas, la bactérie persiste plusieurs semaines dans d'autres plusieurs mois. Au cours de certaines infections, les isolats ont un aspect différent. Ces aspect différent correspond à l'arrêt soit de la synthèse d'AC-Hly, soit de la synthèse de tous les facteurs codés par les gènes vag.

Les inventeurs ont observé de façon tout à fait intéressante dans le cadre de la recherche d'une protection efficace contre la maladie causée par une infection due à des Bordetelles et contre la persistance chez l'hôte de ces bactéries, que les extraits bactériens selon l'invention provoquent une réponse immunitaire humorale et une réponse immunitaire cellulaire.

Ils ont montré qu'après infection par B. bronchiseptica, il y a induction d'une immunité humorale et d'une immunité cellulaire comme dans le cas d'une infection à B. pertussis. De plus, après vaccination avec de l'AC-Hly purifiée, il y a induction d'une immunité de type humoral et cellulaire semblable à celle induite après infection ou réinfection.

L'invention a aussi pour objet des compositions vaccinantes comprenant, à titre de principe actif, une composition immunogène répondant à l'une des définitions données dans les pages qui précèdent, en association avec un véhicule pharmaceutique acceptable et le cas échéant avec un adjuvant.

Comme les vaccins coquelucheux actuellement disponibles sur le marché, le vaccin selon l'invention peut être associé à d'autres principes actifs vaccinants, par exemple ceux du vaccin contre la diphtérie, la polio ou des maladies à Haemophilus ou de façon générale à tout constituant immunogène, par exemple une toxine ou un agent pathogène inactivé déterminé.

Une composition vaccinante selon l'invention peut être spécifique d'espèce et en conséquence capable d'induire une protection contre B. pertussis ou B. parapertussis ou B. bronchiseptica. Au contraire, il peut s'agir d'un mélange comprenant à titre de principe actif une composition immunogène contre B. pertussis telle que définie précédemment et une composition immunogène contre B. parapertussis.

Selon un autre mode de réalisation de l'invention, la composition vaccinante contient les compositions immunogènes élaborées selon l'invention contre B. pertussis et contre B. parapertussis et contre B. bronchiseptica.

Un vaccin particulièrement préféré contre B. pertussis est caractérisé en ce qu'il comprend à titre de principe actif, un "extrait urée vag" de B. pertussis et un "extrait urée vrg" de B. pertussis, la souche de B. pertussis utilisée pour préparer ces extraits étant de préférence la souche HAV déposée à la CNCM sous le n° I-1485.

Un vaccin particulièrement préféré contre B. parapertussis est caractérisé en ce qu'il comprend à titre de principe actif, un "extrait urée vag" de B. parapertussis et un "extrait urée vrg" de B. parapertussis, la souche de B. parapertussis utilisée pour préparer ces extraits étant de préférence la souche n° 1 déposée à la CNCM sous le n° I-1498.

Un vaccin particulièrement préféré contre B. bronchiseptica est caractérisé *en* ce qu'il comprend à titre de principe actif, un "extrait urée vag" de B. bronchiseptica et un "extrait urée vrg" de B. bronchiseptica, la souche de B. bronchiseptica utilisée pour préparer ces extraits étant dé préférence la souche 973S déposée à la CNCM sous le n° I-858.

Toute composition vaccinante comprenant un mélange des extraits urée vag et des extraits urée vrg de B. pertussis et/ou de B. parapertussis et/ou de B. bronchiseptica entre également dans le cadre de l'invention.

Le cas échéant, les extraits urée vag et vrg mis en oeuvre sont préparés à partir de plusieurs isolats des Bordetella utilisés.

L'invention vise aussi les extraits bactériens de type "extraits urée" tels qu'obtenus par mise en oeuvre du procédé décrit en détail dans la partie expérimentale.

Entrent également dans le cadre de l'invention, des compositions immunogènes ou des compositions vaccinantes dans lesquelles l'AC-Hly est présente sous forme pure ou en ce qu'elle est remplacée par un polypeptide comprenant sa partie C-terminale et/ou un polypeptide comprenant sa partie interne.

L'AC-Hly peut être isolée à partir de Bordetella ou préparée par exemple par les techniques du génie génétique.

L'invention a également pour objet des anticorps tels que produits chez un hôte, auquel on a préalablement administré des compositions immunogènes ou vaccinantes telles que décrites ci-dessus ou un extrait bactérien.

FIGURES

Figure 1 : Détection d'anticorps sériques spécifiques après immunisation par un extrait urée vag Bordetella bronchiseptica, inactivé ou non.

Figure 2: Lymphoprolifération: Vaccin extrait urée vag Bordetella bronchiseptica.

Figure 3: Infection respiratoire à Bordetella bronchisentica.

Figure 4: Evolution de l'infection et de la ré-infection à B. bronchiseptica chez des souris primo-infectées avec ces bactéries ou vaccinées avec ces bactéries ou avec de l'AC-Hly purifiée.

Figure 5: Virulence comparée de B. bronchiseptica 973S et d'un mutant B. bronchiseptica aflagellé (973S fla-).

Partie expérimentale

En raison de l'ensemble des résultats exposés ci-dessus, le modèle suivant est proposé pour expliquer le devenir de la Bordetelle chez l'hôte :

Etape 1. Pour initier l'infection, la bactérie exprimerait les gènes vag, les adhésines tels la FHA, les AGG et la PRN permettant ainsi l'adhésion de la bactérie sur les cellules cibles et les toxines telles l'AC-Hly et la PTX permettant ainsi la destruction de la première ligne de défense de l'hôte.

Etape 2. Le but de la bactérie étant de persister et non de tuer l'hôte, elle ne peut continuer à synthétiser de façon continue des toxines comme l'AC-Hly ou la PTX qui détruisent les défenses de l'hôte, en particulier, macrophages et lymphocytes. Elle arrêterait donc la synthèse de ses toxines mais continuerait à synthétiser ses adhésines.

Etape 3. Afin de persister et d'échapper aux défenses immunitaires de l'hôte qui se seraient développées contre les adhésines, la bactérie arrêterait la synthèse des adhésines et exprimerait alors les protéines codées par les gènes vrg. Les arguments que nous avons en faveur de ce modèle sont que:

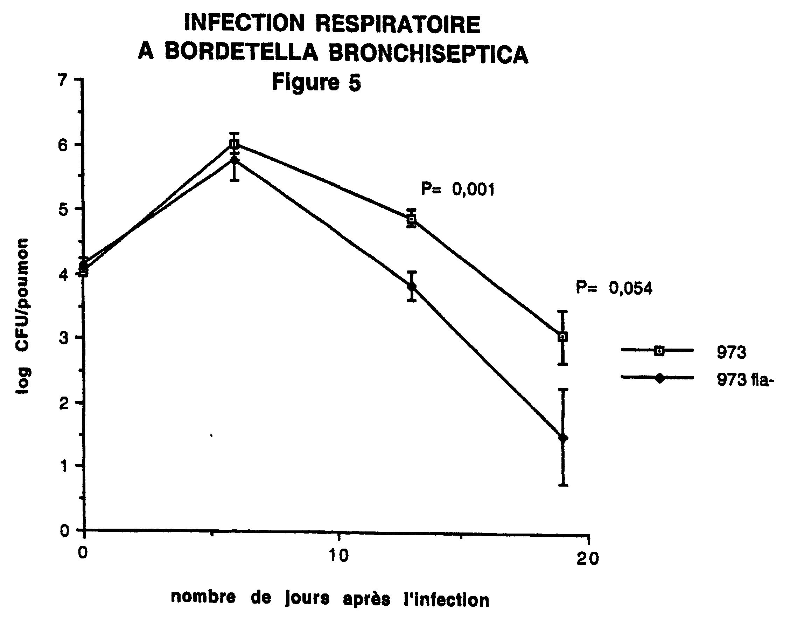

- des souches de Bordetelles exprimant soit tous les gènes vag soit les gènes vag codant pour les adhésines uniquement soit aucun gène vag sont isolées au cours de la maladie,
- la capacité d'induire la létalité par les différents isolats cliniques de B. bronchiseptica varie en fonction de la quantité d'AC-Hly exprimée et sécrétée
- après infection de cobayes avec une culture de B. bronchiseptica exprimant les gènes vag, des anticorps dirigés contre les flagelles, produits de gènes vrg, suggérant que la bactérie fait varier la synthèse de ses différents facteurs au cours de l'infection, peuvent être détectés.
- un mutant de B. bronchiseptica exprimant tous les gènes vag mais n'exprimant pas de flagelline (produit vrg, B. J. Akerley, D.M. Monack, A. Falkow, J.H. Miller.J.Bacteriol. 1992. 174, 980-990) et étant donc immobile, construit au laboratoire par mutagénèse insertionnelle, est, d'après nos résultats préliminaires, moins virulent dans le modèle murin d'infection respiratoire : la DL 50 (dose létale 50) de la souche parentale, B. bronchiseptica 973 S, est d'environ $10^7$ bactéries et celle du mutant fla- est d'environ $5.10^7$ bactéries. Par ailleurs, il semble que ce mutant soit éliminé de l'appareil respiratoire plus rapidement que la souche parentale, suggérant l'importance du flagelle pour la persistance de B. bronchiseptica chez l'hôte (Figure 5).

**Vaccin acellulaire efficace contre la maladie et l'infection induites par Bordetella bronchiseptica.**

En accord avec le modèle précédent, le vaccin acellulaire selon l'invention a donc été défini pour protéger non seulement contre l'effet toxique dû aux différents facteurs mais aussi contre la persistance de la bactérie. Ce vaccin pour être efficace est constitué des facteurs nécessaires à la bactérie pour adhérer, se multiplier mais aussi persister extra ou intracellulairement, donc être constitué à la fois des produits des gènes vag mais aussi des gènes vrg.

Pour limiter le coût résultant de la préparation d'un vaccin constitué de tous ces facteurs purifiés, un vaccin acellulaire d'un coût moindre est celui constitué d'un mélange d'"extraits urée" obtenus à partir de bactéries exprimant les gènes vag et à partir de bactéries exprimant les gènes vrg.

Un "extrait urée" est composé d'un mélange de protéines exprimées à la surface de la bactérie et qui sont séparées de la bactérie après incubation de celle-ci avec de l'urée 5M. L'"extrait urée vag" de B. bronchiseptica contient entre autres protéines l'AC-Hly, la FHA, la PRN et le LPS (endotoxine) et l'"extrait urée vrg" contient, plusieurs protéines non encore caractérisées, les flagelles et le LPS.

Dans le cas des Bordetelloses animales, le seul agent de la maladie est B. bronchiseptica et le vaccin est constitué d'un mélange d'"extrait urée vag" et d'"extrait urée vrg" préparés à partir d'un ou de plusieurs isolats de B. bronchiseptica, si la diversité entre les souches de B. bronchiseptica, mise en évidence grâce à l'utilisation de la technique d'électrophorèse en champ pulsé, se situe au niveau des protéines constituant ces extraits urée. Il sera important de tester la nécessité d'inactiver les protéines constituants ces extraits, en particulier l'AC-Hly. Par ailleurs, il faudra tester la nécessité de conserver le LPS dans ces extraits.

Vaccin acellulaire efficace contre la maladie et l'infection induites par Bordetella pertussis et Bordetella parapertussis.

Dans le cas des Bordetelloses humaines B. pertussis, B. parapertussis et B. bronchiseptica peuvent être les agents de la maladie. Le vaccin pourrait alors être constitué de suspensions bactériennes exprimant les gènes vag et de suspensions bactériennes exprimant les gènes vrg des représentants des trois espèces de Bordetelles (ce vaccin serait alors constitué de six suspensions bactériennes).

Ces suspensions bactériennes pourraient être obtenues à partir de bactéries dont les toxines ont été inactivées par génie génétique afin de diminuer les effets secondaires dus à la vaccination avec des bactéries entières.

1. Préparation d'extrait urée vag et vrg

1.1. Extrait urée vag

Les extraits urée vag sont préparés à partir de bactéries du genre Bordetella (espèces pertussis, parapertussis ou bronchiseptica) phase I, c'est à dire des bactéries exprimant les gènes vag. Les protéines exprimées à partir des gènes vag sont entre autres : l'adényl cyclase-hémolysine (AC-Hly), la toxine de pertussis (PTX) et les agglutinogènes (AGG).

Le protocole de préparation de ces extraits urée est le même pour les 3 espèces bactériennes.

1.2. Extráit urée vrg

Les extraits urée vrg sont préparés à partir des 3 mêmes espèces bactériennes phase IV c'est à dire à partir de bactéries n'exprimant plus aucun gène vag mais exprimant les gènes vrg. Les produits de ces gènes ne sont pas encore bien caractérisés, à l'exception des flagelles de B. bronchiseptica. Les extraits urée vrg se préparent comme les extraits urée vag.

2. Résultats

2.1. Préparation d'extrait urée vag à partir de B. bronchiseptica 973 phase I

2.1.1. Culture de Bordetella bronchiseptica Phase I

- Cultiver les bactéries phase I sur milieu solide Bordet Gengou au sang (BGS ; décrit plus loin) pendant 48 heures de façon à obtenir des colonies isolées hémolytiques.
- Resuspendre ensuite quelques colonies dans du milieu Stainer liquide (décrit plus loin) et étaler 100 $\mu$l de cette suspension sur milieu Stainer solide (CSM ; décrit plus loins).
- Après 24-36 heures de cultures à 36°C, resuspendre les bactéries dans du milieu Stainer liquide et ensemencer une fiole de 2 l contenant 200 ml de milieu Stainer liquide de telle sorte que la densité optique à 650 nm soit de 0,05.
- Après ensemencement, cultiver 18-20 heures à 36°C sous agitation douce de façon à obtenir une densité optique à 650 nm de 1-1,2.
- Doser l'activité adényl cyclase de la suspension bactérienne ainsi obtenue sans la diluer (décrit plus loin). Si celle-ci est comprise entre 30 et 100 U/ml, l'extrait urée peut être préparé.

Pour la préparation des extraits urée vrg, des souches de Bordetella phase IV sont utilisées et cultivées de la même façon que les souches phase I, à l'exception du fait que le milieu Stainer dont il est question ci-dessus, est complété par du sulfate de magnésium 50 mM. Le cas échéant, le sulfate de magnésium est remplacé par d'autres régulateurs, tels que décrits dans la publication de Melton A., Weiss A., Inf. Imm. 1993, 61:807-815.

2.1.2. Préparation de l'extrait urée vag

- Centrifuger la suspension bactérienne 30 minutes à 5000 g à 4°C.
- Resuspendre le culot bactérien dans de l'urée 5M préparée dans du tampon PBS (décrit plus loin) à raison d'un volume égal à 5 fois le poids humide du culot bactérien.
- Laisser sous agitation durant 1 heure à 4°C puis centrifuger 40 minutes à 40000 g à 4°C,
- Conserver le surnageant à -80°C jusqu'à utilisation après avoir vérifié la présence d'adényl cyclase hémolysine, hémagglutinine filamenteuse et pertactine dans l'extrait à l'aide d'anticorps spécifiques de ces facteurs.

2.1.3.Inactivation de l'extrait urée vag

- Après élimination de l'urée, par passage sur colonne G25, l'extrait urée vag est dilué en PBS de façon à obtenir une concentration protéique de 300 $\mu$g/ml.
- Ajouter goutte à goutte un volume de glutaraldéhyde à 2,5% de façon à obtenir une concentration finale de 0,05%.
- Laisser 2 heures à température ambiante en mélangeant régulièrement.
- Arrêter la réaction par addition de lysine (concentration finale 0,02M).
- Après 2 heures à température ambiante, l'extrait urée est adsorbé sur hydroxyde d'alumine préparé en PBS (1 mg/ml), pendant une nuit à 4°C sous agitation douce.

Le vaccin est alors prêt pour l'immunisation de l'animal à raison de 10 à 20 $\mu$g/injection (décrit plus loin).

2.2. Efficacité protectrice de l'extrait uréee vag B. bronchiseptica dans le modèle murin d'infection respiratoire.

2.2.1. Immunisation

- Immuniser deux groupes de souris à 14 jours d'intervalle avec soit 250 µl d'hydroxyde d'alumine à 1 mg/ml soit 250 µl d'extrait urée vag adsorbé sur hydroxyde d'alumine.

2.2.2. Réponse humorale

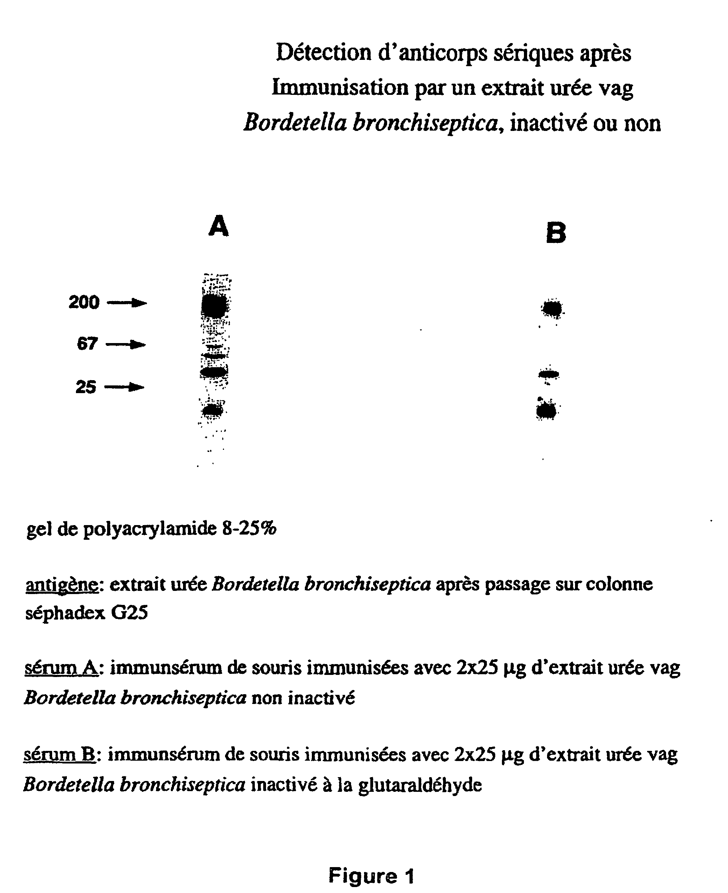

- 7 jours après la 2ème immunisation, vérifier la présence d'anticorps dans le sérum des souris vis à vis X des protéines de l'extrait urée vag par la technique de Western Blot (figure 1). Les anticorps produits sont de type IgG incluant des immunoglobulines spécifiques de type IgG2a, de type IgM IgA.

2.2.3. Réponse cellulaire

La réponse induite après immunisation avec la bactérie entière a été comparée à celle obtenue après immunisation avec l'extrait urée vag inactivé ou non ou avec un produit vag purifié, l'AC-Hly.

La réponse cellulaire est étudiée 7 jours après la deuxième immunisation chez une partie des souris.

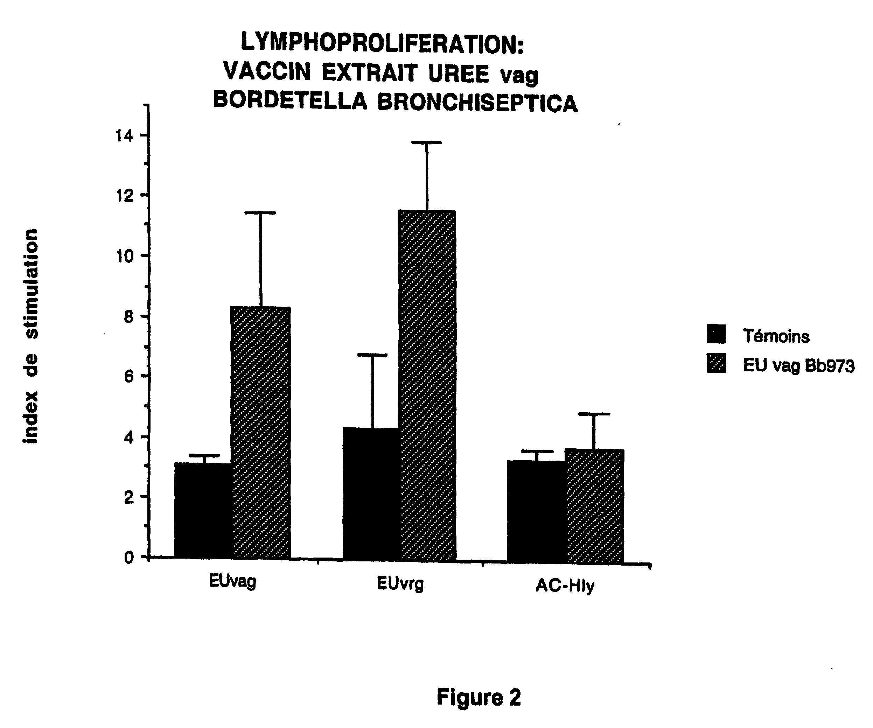

Dans la figure 2, les splénocytes de souris immunisées avec l'extrait urée vag non inactivé ($2\times 10^6$ cellules par ml) sont mis en culture avec soit l'extrait urée vag (EU vag), soit l'extrait urée vrg (EU vrg), soit l'AC-Hly à une concentration protéique finale de 1 µg/ml (d'après le protocole de Redhead et al).

Une réponse proliférative est obtenue avec l'extrait urée vag et l'extrait urée vrg. Des cytokines caractéristiques de cellules Th1 ou Th2 sont synthétisées.

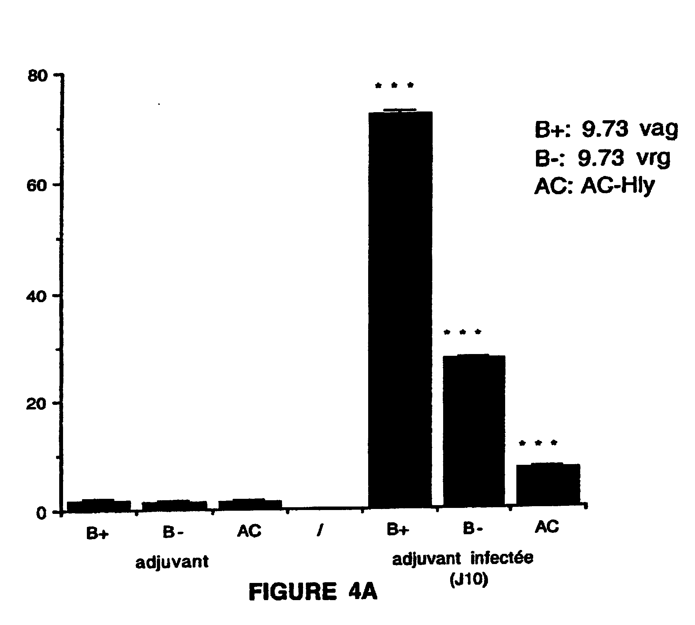

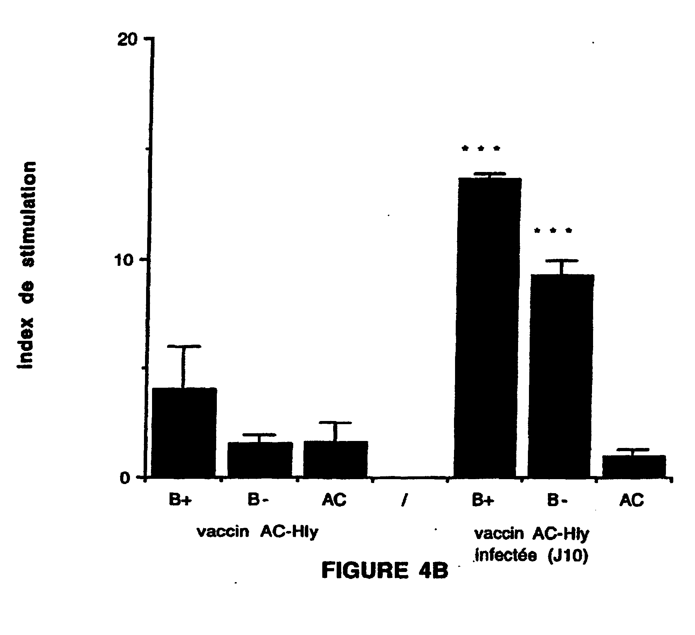

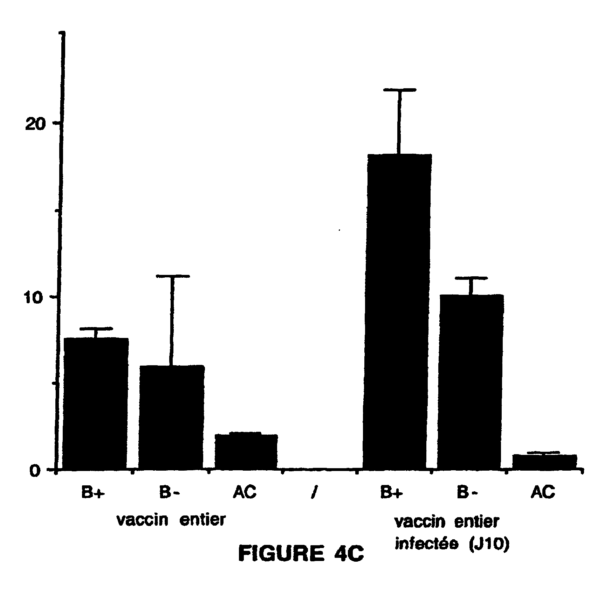

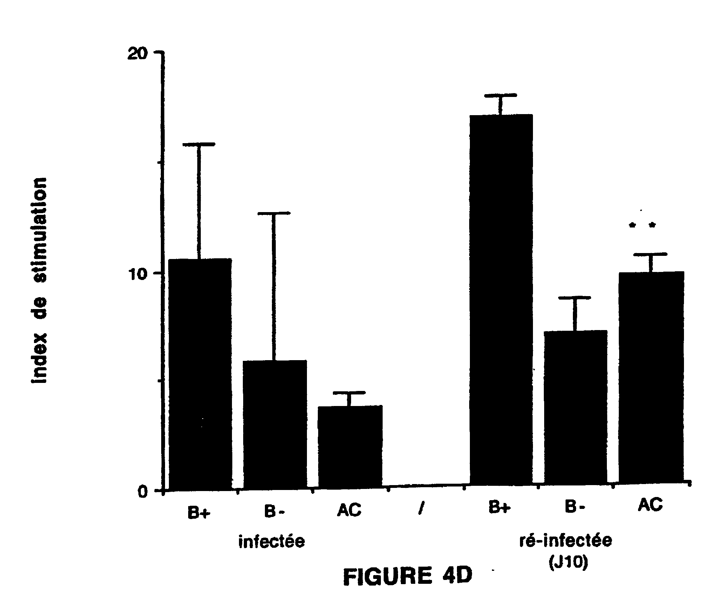

La figure 4 représente l'analyse de l'Immunité à médiation cellulaire en réponse aux produits d'expression des gènes *vag*, aux produits d'expression des gènes *vrg* et à l'AC-Hly soit suite à une infection (fig. 4A), soit suite à une vaccination et une infection (vaccination par AC-Hly, fig. 4B, et vaccination par vaccin entier, fig. 4C), soit suite à une infection et une reinfection (fig. 4D).

Au cours de la primo-infection, on observe une réponse importante induite contre ces différents antigènes. Cette réponse est plus faible chez les souris vaccinées ou réinfectées car il s'agit dans ce cas d'une réponse secondaire faisant intervenir les lymphocytes T immuns spécifiquement sensibilisés lors de la vaccination ou de la primo-infection.

2.2.4 Epreuve

Le reste des souris est infecté par une dose subléthale de bactéries.

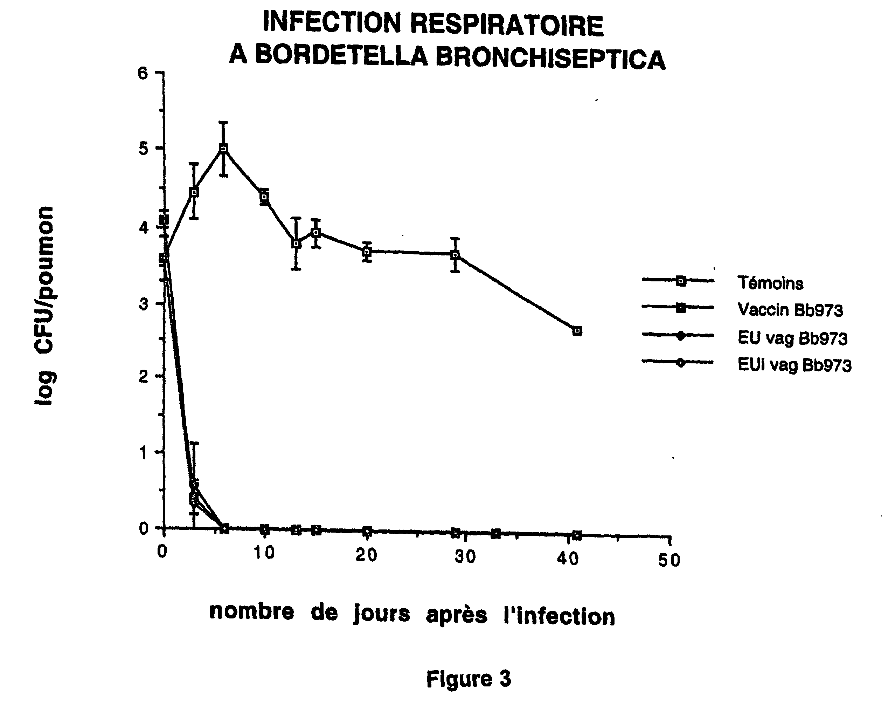

- 14 jours après la 2è immunisation, les 2 groupes de souris sont infectés par voie intranasale avec 50 µl d'une suspension bactérienne de Bordetella bronchiseptica Phase I cultivée en milieu Stainer comme décrit précédemment.
- A $J_0$, $J_3$, $J_6$, $J_{10}$, $J_{12}$, $J_{15}$, $J_{30}$, $J_{40}$, 3 ou 4 souris de chaque groupe sont sacrifiées et une numération des bactéries *est* faite au niveau du poumon.
- Les résultats obtenus sont semblables à ceux obtenus après vaccination avec un vaccin composé de bactéries entières (figure 3).

Figure 1 : Détection d'anticorps sériques spécifiques après immunisation par un extrait urée vag Bordetella bronchiseptica, inactivé ou non gel de polyacrylamide 8-25%

antigène: extrait urée Bordetella bronchiseptica après passage sur colonne séphadex G25

sérum A: immunsérum de souris immunisées avec 2x25 µg d'extrait urée vag Bordetella bronchiseptica non inactivé

sérum B: immunsérum de souris immunisées avec 2x25 µg d'extrait urée vag Bordetella bronchiseptica inactivé à la glutaraldéhyde.

Description des milieux de culture

Milieu Bordet Gengou deshydraté

| Composition: | | |
|---|---|---|
| | *1 litre* | *5 litres* |
| *Milieu Bordet Gengou* | *30 g* | *150 g* |
| *Glycérol* | *10 ml* | *50 ml* |
| *Eau pyrolysée* | *1 litre* | *5 litres* |
| *Ajuster le pH à 7,4* | | |

- Faire chauffer
- Autoclaver 15 minutes à 120°
- Conserver à 4°C

Au moment de l'emploi :

Le Bordet Gengou est enrichi avec 15 à 20% de sang de mouton ou de cheval. Faire fondre les tubes et les maintenir fondus à 54°C. Rajouter 2,5 ml de sang de mouton dans chaque tube, stérilement. Verser le contenu du tube dans une boîte de pétri stérile.

Remarque:

Pour la recherche de Bordetella pertussis à partir de prélèvement nasopharyngé, utiliser des boites fraîches (maximum 7 jours à 4°C)

Milieu Gélose CSM

Pour préparer 2 litres d'une solution concentrée 10 fois :

| | | |
|---|---|---|
| Sodium Hydrogénoglutamate | (Réf. Prolabo n°27872.298) | 107g |
| L-Proline | (Réf. Merck n°7434) | 2,4g |
| NaCl | (Réf. Prolabo n°27810.295 | 25g |
| H2PO4 | (Réf. Prolabo n°26926.298) | 5g |
| KCl | (Réf. Prolabo n°26759.291) | 2g |
| MgCl2 | (Réf. Prolabo n°25108.295) | 1g |
| Tris-base | (Réf. Merck n°8382.2500) | 15,2g |
| Cesamino Acids | (Réf. Difco n° 0288-01-2) | 5g |
| Solution de CaCl2 à 1% dans l'eau pyrolysée | (Réf. Prolabo n°22317.297 | 20 ml |
| Eau pyrolysée | QSP | 1 litre |

Dissoudre les différents constituants dans une partie du volume d'eau final. Ajuster le pH à 7,4 à l'aide d'acide chlorydrique. Compléter au volume final et conserver à -20°C.

- Au moment de l'emploi, mélanger:

- 100 ml de la solution concentrée 10 fois
- 900 ml d'eau pyrolysée
- 1 g de (2,6-0-Dimethyl) cyclodextrine référence Aldrich n° 51166-71-3
- 15 g de Bacto agar référence Difco n°0140-01

Répartir par fractions de 20 ml en tube de verre Stériliser et ajouter le complément stérile.

- Solution de complément :
- Mélanger :

- 1 ml de solution de complément concentrée 10 fois
- 100 mg de glutathion référence Merck n° 4090
- 9 ml d'eau pyrolysée

Filtrer cette solution sur filtre millex 0,22 μm
Ajouter 200 μl de cette solution à 1 tube de 20 ml de milieu

### Milieu de culture Stainer

### A. Milieu de Base

Pour préparer 2 litres d'une solution concentrée 10 fois :

| | | |
|---|---|---|
| Sodium Hydrogénoglutamate | (Réf. Prolabo n°27872.298) | 214,0g |
| L-Proline | (Réf. Merck n°7434) | 4,8g |
| NaCl | (Réf. Prolabo n°27810.295) | 50,0g |
| H2PO4 | (Réf. Prolabo n°26926.298) | 10,0g |
| KCl | (Réf. Prolabo n°26759.291) | 4,0g |
| MgCl2 | (Réf. Prolabo n°25108.295) | 2,0g |
| Tris-base | (Réf. Merck n°8382.2500) | 30,5g |
| Solution de CaCl2 à 1% dans l'eau pyrolyses | (Réf. Prolabo n°22317.297 | 40 ml |
| Eau pyrolyses | QSP | 2 litres |

Dissoudre les différents constituants dans une partie du volume d'eau final. Ajuster le pH à 7,6 à l'aide d'acide chlorydrique. Compléter au volume final et répartir cette solution concentrée qui peut être conservée à -20°C pendant plusieurs semaines.
Au moment de l'emploi, diluer le milieu, le stériliser à 120°C pendant 15 minutes puis ajouter le complément stérilisé par filtration.

### B. Solution de complément

Pour préparer 200 ml d'une solution concentrée 10 fois :

- L-Cystine (Réf. Prolabo n°23260.184) 8g
- HCl concentré 20ml

Dissoudre. Sur cette préparation, verser le mélange suivant préalablement dissout :

| | | |
|---|---|---|
| FeSO4, 7H2O | (Réf. Prolabo n°24244.232) | 2g |
| Acide L(+) ascorbique | (Réf. Prolabo n°20155.237) | 4g |
| Acide nicotinique | (Réf. Merck n°6817) | 0,8g |
| Eau pyrolysée | | 120ml |

Compléter à 200 ml avec de l'eau pyrolysée, répartir la solution par fractions de 1, 2, 3 ou 4 ml et congeler à -20°C.
Au moment de l'emploi, diluer la solution 10 fois dans l'eau pyrolysée et ajouter :

le glutathion (Réf. Merck n°4090)....100mg/10ml de complément dilué, stériliser cette solution par filtration (filtre Millex 0,22 μm à usage unique) et ajouter 1 ml de solution stérile à 100 ml de milieu de base stérile.

### Dosage Activité cyclase

Préparation des solutions:

| Mix* | | |
|---|---|---|
| Tris 1M pH8 | 4,15 ml | (83,3 mM final) |
| MgCl2 0,1 M | 5ml | (10 mM final) |
| AMPc 0,34 M | 29μl | (0,2 mM final) |
| eau | 40,5 ml | |
| +200μl AMPc*H$^3$ de façon à avoir 2.14$^4$ cpm/60 μl | | |

Après avoir homogénéisé la solution, répartir dans des tubes par 4 ml et conserver à -20°C.

1) Mélange BSA-Calmoduline:

| BSA 10mg/ml | 1xμl |
|---|---|
| Calmoduline 2 μl | 1xμl |
| eau | 2xμl |

La BSA est diluée dans l'eau et la calmoduline est diluée dans du Tris.
ATP*:
ATP froid 20mM
+ATP*P$^{32}$
      de façon à avoir environ 8.10$^5$ cpm/10μl (Jour 0)
      on peut l'utiliser jusqu'à 5.10$^5$ cpm/10μl

2) Réalisation du dosage:

.    Important: faire tous les dosages en double, y compris les témoins.
.    Dans chacun des tubes en verre préalablement étiquetés, mélanger:

   60μl de Mix*
   20 μl de BSA-CaM-eau
   10 μl d'échantillon (exemple : suspension bactérienne DO=1, ou diluer avec Tris 50 mM pH8)

.    Placer les tubes dans un bain à 30°C puis ajouter 10μl d'ATP* et laisser pendant 10 minutes précisément.
.    Ajouter 200μl d'HCl 0,5 M pour arrêter la réaction.
.    Plonger les tubes dans un bain marie bouillant pendant 5 minutes puis ajouter 200μl d'imidazole 1,5M. (Etape non nécessaire).
.    Laisser refroidir puis déposer sur colonne d'alumine (1 gramme par colonne). Eluer avec 3 ml d'imidazole 10 mM.
.    Recueillir l'éluat dans 10 ml de scintillant et compter le nombre de cpm H$^3$ pour calculer le rendement de la colonne et le nombre de cpm P$^{32}$ pour calculer l'activité cyclase. (Attention au "quenching").
.    Faire un "blanc": les 10μl d'échantillon sont remplacés par 10μl d'eau.
.    Deux "activités totales":

   1) H$^3$: 3ml d'imidazole sont élués d'une colonne et recueillis dans des fioles contenant 10 ml de scintillant et 60μl de Mix.
   2) P$^{32}$: idem mais les fioles contiennent 10 ml de scintillant et 10μl d'ATP-P$^{32}$.

3) Calculs

1) Calcul du rendement de chaque colonne:

$$\frac{\text{cpm 32P x cpm 3H totaux}}{\text{cpm 3 H de l'échantillon}} = X \text{ cpm..... } X', X'', X'''...$$

2) Calcul du blanc:

identique = Y cpm

3) Z= nombre de cpm totaux pour chaque échantilon
X cpm - Ycpm = Z cpm..... Z', Z'', Z'''....

4)

$$\text{cpm } P^{32} \text{ totaux} \rightarrow 200 \text{ nmoles d'ATP}$$

$$Z \text{ cpm} \rightarrow W \text{ nmoles}$$

W nmoles pour v µl enzyme
T= temps en minutes

$$N \text{ nmoles/minute/ml} = \frac{W \times \text{facteur de dilution (100 si 10µl}}{10 \text{ (temps)}}$$

Si l'échantillon a été dilué avant le dosage, multiplier par le facteur de dilution.

$$G \text{ nmoles/minute/mg} = \frac{N}{mg}$$

**Préparation de l'ATP et de l'AMPc:**

ATP froid: une fois mis en suspension, utiliser tout de suite ou congeler à -20°C. Ne pas décongeler et congeler plusieurs fois... très fragile! Ne pas conserver longtemps.
Préparer 5 ml d'ATP 20 mM: peser environ 70 mg et ajouter 5 ml de Tris 50 mM pH7. De cet ATP dilué au 1/1000ème, mesurer la D.O. très précisément à 260 nm. Calculer la concentration exacte sachant que:

$$1 \text{ mM} \rightarrow D_{260}=15,4$$

$$\text{ou } 10^{-5} \text{ M } \_\_\_> DO_{260}=0,154$$

AMPc froid: Préparer comme l'ATP, il faut seulement dissoudre 32,9 mg/10 ml (on peut chauffer un peu pour dissoudre). De même, déterminer la concentration exacte en mesurant la DO à 260 nm, la solution étant diluée au 1/500ème.

$$10^{-5} \text{ M} \rightarrow DO_{260}=0,154$$

Références des produits utilisés

Tris: réf. 8382 chez Merck
BSA, albumine bovine: réf. A-4503 chez Sigma
Calmoduline ou Phosphodiesterase 3':5'-cyclic nucleotide-activator: réf. P 2277 chez Sigma
Imidazole: réf. I-0152 chez Sigma
Scintillant: Optiphase "HiSafe 3', chez LKB-Pharmacia
ATP froid: réf A-2383 (PM=551,1) chez Sigma
ATP P[32] ; Amersham, 500 µCi, réf. PB 200 = alpha P[32]
AMPc froid: A-9501 chez Sigma
AMP H[3]: Amersham [la plus petite dose (10µCi)]

**PREPARATION D'UN VACCIN ACELLULAIRE**

Bordetella bronchiseptica

Adsorption sur hydroxyde d'alumine

.   Référence hydroxyde d'alumine $Al(OH)_3$: Alhydrogel 3 % $Al(OH)_3$ équivalent à 2% $Al_2O_3$ (Superfos Biosector als Denmark)

.   Tampon de dilution: tampon phosphate-NaCl 0,15M pH=6,8 <u>Formule</u> (concentré 10 fois): $PO_4$ 0,1M (soit $Na_2HPO_4$, $_2H_2O$ 0,07M
    et $NaH_2 PO_4$, $H_2O$, 0,03M)
    NaCl 1,5M
    ph=6,8 diluer 10 fois dans de l'eau pyrolysée au moment de l'utilisation.

.   Lavage de l'hydroxyde d'alumine:

    5 ml d'$Al(OH)_3$ concentré sont lavés dans 50 ml de tampon x 1 à 2 reprises.

Après le deuxième lavage, les 5 ml d'$Al(OH)_3$ lavés sont repris dans un volume total de 50 ml, soit de l'Al $(OH)_3$ concentré 2 fois à la finale (concentration finale : 2 mg/ml).

L'adjuvant ainsi préparé est mélangé volume à volume avec la fraction vaccinale et mis à adsorber pendant une nuit à 4°C sous agitation. La concentration finale d'$Al(OH)_3$ est de 1 mg/ml dans le vaccin prêt à être injecté à l'animal.

**Revendications**

**1.** Composition immunogène, **caractérisée en ce qu'**elle comprend une protéine adénylcyclase-hémolysine (AC-Hly) ou une partie immunogène de cette AC-Hly, d'une souche de Bordetella choisie parmi <u>B. pertussis</u>, <u>B. parapertussis</u> ou <u>B. bronchiseptica</u> et **en ce qu'**elle comprend en outre un extrait bactérien contenant les produits d'expression des gènes <u>vrg</u> d'une souche de <u>Bordetella</u> choisie parmi <u>B. pertussis, B. parapertussis</u> ou <u>B. bron- chiseptica</u> ou une partie de ces produits d'expression, suffisante pour induire une réponse immunitaire chez un hôte auquel l'extrait serait administré.

**2.** Composition immunogène selon la revendication 1 **caractérisée en ce qu'**elle comprend en outre une ou plusieurs adhésines ou toxines de <u>B. pertussis</u>, <u>B.parapertussis</u> ou <u>B. bronchiseptica</u>, choisie(s) parmi la FHA, les AGG ou la PRN et la PTX.

**3.** Composition immunogène selon la revendication 1 ou la revendication 2 **caractérisée en ce qu'**elle comprend la toxine PTX de <u>B. pertussis</u>, la toxine AC-Hly de <u>B. pertussis</u> et un extrait bactérien contenant les protéines codées par les gènes <u>vrg</u> de <u>B. pertussis</u>, ou une partie de ces protéines, suffisante pour induire une réponse immunitaire chez un hôte auquel l'extrait serait administré.

**4.** Composition immunogène selon la revendication 3, **caractérisée en ce que** la toxine AC-Hly de <u>B. pertussis</u> est contenue dans un extrait bactérien contenant tout ou partie des facteurs de virulence du groupe des adhésines ou des toxines de <u>B. pertussis</u>.

**5.** Composition immunogène selon la revendication 4, **caractérisée en ce que** la toxine AC-Hly de <u>B. pertussis</u> est présente sous la forme d'un extrait urée dit "extrait urée vag".

**6.** Composition immunogène selon l'une quelconque des revendications 1 à 4, **caractérisée en ce que** la toxine AC-Hly de <u>B. pertussis</u> est présente sous la forme d'un extrait urée dit "extrait urée vag" et **en ce que** l'extrait bactérien contenant tout ou partie des protéines codées par les gènes <u>vrg</u> de <u>B. pertussis</u> est également un extrait urée dit "extrait urée vrg".

**7.** Composition immunogène selon la revendication 1 ou la revendication 2. **caractérisée en ce qu'**elle comprend la toxine AC-Hly de B. parapertussis et un extrait bactérien contenant tout ou partie des protéines codées par les gènes vrg de B. parapertussis.

**8.** Composition immunogène selon la revendication 7, **caractérisée en ce que** la toxine AC-Hly de B. parapertussis est contenue dans un extrait bactérien contenant tout ou partie des facteurs de virulence du groupe des adhésines ou des toxines de B. parapertussis.

**9.** Composition immunogène selon la revendication 8, **caractérisée en ce que** la toxine AC-Hly de B. parapertussis est présente sous la forme d'un extrait urée dit "extrait urée vag".

**10.** Composition immunogène selon l'une quelconque des revendications 1, 2, 7 ou 8, **caractérisée en ce que** la toxine AC-Hly de B. parapertussis est présente sous la forme d'un extrait urée dit "extrait urée vag" et **en ce que** l'extrait bactérien contenant tout ou partie des protéines codées par les gènes vrg de B. parapertussis est également un extrait urée dit "extrait urée vrg".

**11.** Composition immunogène selon la revendication 1 ou la revendication 2 **caractérisée en ce qu'**elle comprend la toxine AC-Hly de B. bronchiseptica et un extrait bactérien contenant tout ou partie des protéines codées par les gènes vrg de B. bronchiseptica.

**12.** Composition immunogène selon la revendication 11, **caractérisée en ce que** la toxine AC-Hly de B. bronchiseptica est contenue dans un extrait bactérien contenant tout ou partie des facteurs de virulence du groupe des adhésines ou des toxines de B. bronchiseptica.

**13.** Composition immunogène selon la revendication 12, **caractérisée en ce que** la toxine AC-Hly de B. bronchiseptica est présente sous la forme d'un extrait urée dit "extrait urée vag".

**14.** Composition immunogène selon l'une quelconque des revendications 1, 2, 11 ou 12, **caractérisée en ce que** la toxine AC-Hly de B. bronchiseptica est présente sous la forme d'un extrait urée dit "extrait urée vag" et **en ce que** l'extrait bactérien contenant tout ou partie des protéines codées par les gènes vrg de B. bronchiseptica est également un extrait urée dit "extrait urée vrg".

**15.** Composition immunogène selon l'une quelconque des revendications 1,2 ou 11 à 14, **caractérisée en ce qu'**elle comprend en outre des composants polypeptidiques caractéristiques des flagelles de B. bronchiseptica.

**16.** Composition immunogène selon la revendication 1 ou la revendication 2, **caractérisée en ce que** la toxine AC-Hly est sous forme pure.

**17.** Composition immunogène selon la revendication 16, **caractérisée en ce que** la toxine est remplacée par des fragments de l'AC-Hly contenant au moins la partie C-terminale de la toxine et/ou au moins un domaine interne de l'AC-Hly.

**18.** Composition immunogène selon l'une quelconque des revendications 1 à 6, **caractérisée en ce que** la souche de B. pertussis à partir de laquelle sont obtenues la toxine AC-Hly et les protéines exprimées par les gènes vrg, est la souche HAV déposée à la CNCM sous le n° I-1485.

**19.** Composition immunogène selon l'une quelconque des revendications 1, 2 ou 7 à 10, **caractérisée en ce que** la souche de B. parapertussis à partir de laquelle sont obtenues la toxine AC-Hly et les protéines exprimées par les gènes vrg, est la souche n° 1 déposée à la CNCM sous le n° I-1498.

**20.** Composition immunogène selon l'une quelconque des revendications 1, 2, ou 11 à 15, **caractérisée en ce que** la souche de B. bronchiseptica à partir de laquelle sont obtenues la toxine AC-Hly et les protéines exprimées par les gènes vrg, est la souche 973S déposée à la CNCM sous le n° I-858.

**21.** Composition vaccinante **caractérisée en ce qu'**elle comprend à titre de principe actif, une composition immunogène selon l'une quelconque des revendications 1 à 20, en association avec un véhicule pharmaceutiquement acceptable et le cas échéant un adjuvant.

**22.** Composition vaccinante selon la revendication 21, **caractérisée en ce qu'**elle comprend, à titre de principe actif, une composition selon l'une quelconque des revendications 3 à 6 et une composition selon l'une quelconque des revendications 7 à 10.

**23.** Composition vaccinante selon la revendication 22, **caractérisée en ce qu'**elle comprend en outre une composition selon l'une quelconque des revendications 11 à 14.

**24.** Composition vaccinante selon la revendication 21, **caractérisée en ce qu'**elle comprend, à titre de principe actif, un "extrait urée vag" de B. pertussis et un "extrait urée vrg" de B. pertussis, la souche de B. pertussis étant de préférence la souche HAV déposée à la CNCM sous le n° I-1485.

**25.** Composition vaccinante selon la revendication 21, **caractérisée en ce qu'**elle comprend, à titre de principe actif, un "extrait urée vag" de B. parapertussis et un "extrait urée vrg" de B. parapertussis, la souche de B. parapertussis étant de préférence la souche n° 1 déposée à la CNCM sous le n°I-1498.

**26.** Composition vaccinante selon la revendication 21, **caractérisée en ce qu'**elle comprend, à titre de principe actif, un "extrait urée vag" de B. bronchiseptica et un "extrait urée vrg" de B. bronchiseptica, la souche de B. bronchiseptica étant de préférence la souche 973S déposée à la CNCM sous le n°I-858.

**27.** Composition vaccinante selon la revendication 21, **caractérisée en ce qu'**elle comprend, à titre de principe actif, un "extrait urée vag" de B. pertussis et un "extrait urée vrg" de B. pertussis, la souche de B. pertussis étant de préférence la souche HAV déposée à la CNCM sous le n° I-1485 et un "extrait urée vag" de B. parapertussis, la souche de B. parapertussis étant de préférence la souche n° 1 déposée à la CNCM sous le n° I-1498.

**28.** Composition vaccinante selon la revendication 27, **caractérisée en ce qu'**elle comprend en outre un "extrait urée vag" de B. bronchiseptica, la souche de B. bronchiseptica étant de préférence la souche 973S déposée à la CNCM sous le n°I-858.

**29.** Composition vaccinante, **caractérisée en ce qu'**elle comprend, à titre de principe actif, un mélange d'"extraits urée vag" et d'"extraits urée vrg" de souches de B. pertussis et/ou B. parapertussis et/ou B. bronchiseptica.

**30.** Composition vaccinante selon l'une quelconque des revendications 21 à 29, **caractérisée en ce qu'**elle contient en outre, à titre de principe actif, un autre constituant immunogène, par exemple une toxine ou un agent pathogène inactivé.

**31.** Extrait bactérien contenant des facteurs de virulence de Bordetella, susceptible d'entrer dans des compositions vaccinantes, tel qu'obtenu à partir de souches de phase I de B. pertussis, B. parapertussis ou B. bronchiseptica par la mise en oeuvre des étapes suivantes :

- Culture des bactéries sur milieu solide Bordet Gengou au sang (BGS) pendant 48 heures de façon à obtenir des colonies isolées hémolytiques,
- Resuspension de quelques colonies dans du milieu Stainer liquide et étalage de 100 μl de cette suspension sur milieu Stainer solide (CSM),
- Après 24-36 heures de cultures à 36°C, resuspension des bactéries dans du milieu Stainer liquide et ensemencement d'une fiole de 2 l contenant 200 ml de milieu Stainer liquide de telle sorte que la densité optique à 650 nm soit de 0,05,
- Après ensemencement, culture 18-20 heures à 36°C sous agitation douce de façon à obtenir une densité optique à 650 nm de 1-1,2,
- Dosage de l'activité, adényl cyclase de la suspension bactérienne ainsi obtenue sans dilution. Si celle-ci est comprise entre 30 et 100 U/ml, l'extrait urée peut être préparé,
- Centrifugation de la suspension bactérienne 30 minutes à 5000 g à 4°C,
- Resuspension du culot bactérien dans de l'urée 5M préparée dans du tampon PBS à raison d'un volume égal à 5 fois le poids humide du culot bactérien,
- Laissé sous agitation durant 1 heure à 4°C puis centrifugation 40 minutes à 40000 g à 4°C ,
- Conservation du surnageant à -80°C jusqu'à utilisation.

**32.** Extrait bactérien contenant des produits d'expression des gènes vrg, susceptible d'entrer dans des compositions vaccinantes, tel qu'obtenu à partir de souches de phase IV de B. pertussis, B. parapertussis ou B. bronchiseptica par la mise en oeuvre des étapes suivantes :

- Culture des bactéries sur milieu solide Bordet Gengou au sang (BHS) pendant 48 heures de façon à obtenir des colonies isolées hémolytiques,
- Resuspension de quelques colonies dans du milieu Stainer liquide et étalage 100 µl de cette suspension sur milieu Stainer solide (CSM),
- Après 24-36 heures de cultures à 36°C, resuspension des bactéries dans du milieu Stainer liquide et ensemencement d'une fiole de 2 l contenant 200 ml de milieu Stainer liquide de telle sorte que la densité optique à 650 nm soit de 0,05,
- Après ensemencement, culture 18-20 heures à 36°C sous agitation douce de façon à obtenir une densité optique à 650 nm de 1-1,2,
- Centrifugation de la suspension bactérienne 30 minutes à 5000 g à 4°C,
- Resuspension du culot bactérien dans de l'urée 5M préparée dans du tampon PBS à raison d'un volume égal à 5 fois le poids humide du culot bactérien,
- Laissé sous agitation durant 1 heure à 4°C puis centrifugation 40 minutes à 40000 g à 4°C ,
- Conservation du surnageant à -80°C jusqu'à utilisation.

33. Composition immunogène selon l'une quelconque des revendications 1 à 21, **caractérisée en ce que** l'extrait bactérien contenant les produits d'expression des gènes vrg d'une souche de Bordetelle choisie parmi B. pertussis, B. parapertussis ou B. bronchiseptica ou une partie de ces produits d'expression, suffisante pour induire une réponse immunitaire chez un hôte auquel l'extrait serait administré, est remplacé par l'extrait bactérien d'une souche de B. pertussis, B. parapertussis ou B. bronchiseptica, dont le gène bvgS est muté de façon telle que la bactérie exprime essentiellement les gènes vrg au détriment des gènes vag.

34. Souche de Bordetella **caractérisée en ce qu'**il s'agit soit de la souche de B. pertussis HAV déposée à la CNCM sous le n° I-1485, soit de la souche n° 1 de B. parapertussis déposée à la CNCM sous le n° I-1498.

**Patentansprüche**

1. Immunogene Zusammensetzung, **dadurch gekennzeichnet, dass** diese ein Adenylcyclase-Hämolysin (AC-Hly) Protein oder einen immunogenen Anteil dieses AC-Hly eines Bordetellastammes, der ausgewählt ist aus B. pertussis, B. parapertussis oder B. bronchiseptica, umfasst und dadurch, dass diese außerdem einen Bakterienextrakt, welcher die Expressionsprodukte der vrg-Gene eines Bordetellastammes, der ausgewählt ist aus B. pertussis, B. parapertussis oder B. bronchiseptica, oder einen Teil dieser Expressionsprodukte enthält, umfasst, der ausreicht, um bei einem Wirt, welchem der Extrakt verabreicht wird, eine Immunreaktion auszulösen.

2. Immunogene Zusammensetzung nach Anspruch 1, **dadurch gekennzeichnet, dass** diese außerdem ein oder mehrere Adhäsine oder Toxine von B. pertussis, B. parapertussis oder B. bronchiseptica umfasst, das (die) ausgewählt ist (sind) aus dem FHA, den AGG oder dem PRN und dem PTX.

3. Immunogene Zusammensetzung nach Anspruch 1 oder nach Anspruch 2, **dadurch gekennzeichnet, dass** diese das Toxin PTX von B. pertussis, das Toxin AC-Hly von B. pertussis und einen Bakterienextrakt, welcher die Proteine, die durch die vrg-Gene von B. pertussis codiert werden, oder einen Teil dieser Proteine enthält, umfasst, der ausreicht, um bei einem Wirt, welchem der Extrakt verabreicht wird, eine Immunreaktion auszulösen.

4. Immunogene Zusammensetzung nach Anspruch 3, **dadurch gekennzeichnet, dass** das Toxin AC-Hly von B. pertussis in einem Bakterienextrakt enthalten ist, welcher die gesamten oder einen Teil der Virulenzfaktoren der Gruppe der Adhäsine oder der Toxine von B. pertussis enthält.

5. Immunogene Zusammensetzung nach Anspruch 4, **dadurch gekennzeichnet, dass** das Toxin AC-Hly von B. pertussis in Form eines Hamstoffextraktes, genannt "Harnstoffextrakt vag", vorliegt.

6. Immunogene Zusammensetzung nach irgendeinem der Ansprüche 1 bis 4, **dadurch gekennzeichnet, dass** das Toxin AC-Hly von B. pertussis in Form eines Harnstoffextraktes, genannt "Hamstoffextrakt vag", vorliegt und dass der Bakterienextrakt, der die gesamten oder einen Teil der Proteine, die von den vrg-Genen von B. pertussis codiert werden, enthält, gleichfalls ein Harnstoffextrakt, genannt "Hamstoffextrakt vrg", ist.

**7.** Immunogene Zusammensetzung nach Anspruch 1 oder nach Anspruch 2, **dadurch gekennzeichnet, dass** diese das Toxin AC-Hly von B. parapertussis und einen Bakterienextrakt, der die gesamten oder einen Teil der Proteine, die von den vrg-Genen von B. parapertussis codiert werden, enthält, umfasst.

**8.** Immunogene Zusammensetzung nach Anspruch 7, **dadurch gekennzeichnet, dass** das Toxin AC-Hly von B. parapertussis in dem Bakterienextrakt, der die gesamten oder einen Teil der Virulenzfaktoren der Gruppe der Adhäsine oder der Toxine von B. parapertussis enthält, enthalten ist.

**9.** Immunogene Zusammensetzung nach Anspruch 8, **dadurch gekennzeichnet, dass** das Toxin AC-Hly von B. parapertussis in Form eines Harnstoffextraktes, genannt "Harnstoffextrakt vag", vorliegt.

**10.** Immunogene Zusammensetzung nach irgendeinem der Ansprüche 1, 2, 7 oder 8, **dadurch gekennzeichnet, dass** das Toxin AC-Hly von B. parapertussis in Form eines Harnstoffextraktes, genannt "Harnstoffextrakt vag", vorliegt und dass der Bakterienextrakt, welcher die gesamten oder einen Teil der Proteine, die von den vrg-Genen von B. parapertussis codiert werden, enthält, ebenfalls ein Harnstoffextrakt, genannt "Harnstoffextrakt vrg", ist.

**11.** Immunogene Zusammensetzung nach Anspruch 1 oder nach Anspruch 2, **dadurch gekennzeichnet, dass** diese das Toxin AC-Hly von B. bronchiseptica und einen Bakterienextrakt, welcher die gesamten oder einen Teil der Proteine, die von den vrg-Genen von B. bronchiseptica codiert werden, enthält, umfasst.

**12.** Immunogene Zusammensetzung nach Anspruch 11, **dadurch gekennzeichnet, dass** das Toxin AC-Hly von B. bronchiseptica in einem Bakterienextrakt, welcher die gesamten oder einen Teil der Virulenzfaktoren der Gruppe der Adhäsine oder der Toxine von B. bronchiseptica enthält, enthalten ist.

**13.** Immunogene Zusammensetzung nach Anspruch 12, **dadurch gekennzeichnet, dass** das Toxin AC-Hly von B. bronchiseptica in Form eines Harnstoffextraktes, genannt "Harnstoffextrakt vag", vorliegt.

**14.** Immunogene Zusammensetzung nach irgendeinem der Ansprüche 1, 2, 11 oder 12, **dadurch gekennzeichnet, dass** das Toxin AC-Hly von B. bronchiseptica in Form eines Harnstoffextraktes, genannt "Harnstoffextrakt vag", vorliegt, und dass der Bakterienextrakt, welcher die gesamten oder einen Teil der Proteine, die von den vrg-Genen von B. bronchiseptica codiert werden, enthält, ebenfalls ein Harnstoffextrakt, genannt "Harnstoffextrakt vrg", ist.

**15.** Immunogene Zusammensetzung nach irgendeinem der Ansprüche 1, 2 oder 11 bis 14, **dadurch gekennzeichnet, dass** dieser außerdem Polypeptidbestandteile, die für die Flagellen von B. bronchiseptica charakteristisch sind, umfasst.

**16.** Immunogene Zusammensetzung nach Anspruch 1 oder Anspruch 2, **dadurch gekennzeichnet, dass** das Toxin AC-Hly in reiner Form vorliegt.

**17.** Immunogene Zusammensetzung nach Anspruch 16, **dadurch gekennzeichnet, dass** das Toxin durch Fragmente von AC-Hly ersetzt ist, die wenigstens den C-terminalen Anteil des Toxins und/oder wenigstens eine innere Domäne von AC-Hly enthalten.

**18.** Immunogene Zusammensetzung nach irgendeinem der Ansprüche 1 bis 6, **dadurch gekennzeichnet, dass** der Stamm von B. pertussis, ausgehend von welchem das Toxin AC-Hly und die Proteine, die durch die vrg-Gene exprimiert werden, erhalten werden, der Stamm HAV ist, der bei der CNCM unter der Nr. I-1485 hinterlegt wurde.

**19.** Immunogene Zusammensetzung nach irgendeinem der Ansprüche 1, 2 oder 7 bis 10, **dadurch gekennzeichnet, dass** der Stamm von B. parapertussis, ausgehend von welchem das Toxin AC-Hly und die Proteine, die durch die vrg-Gene exprimiert werden, erhalten werden, der Stamm Nr. 1 ist, der bei der CNCM unter der Nr. I-1498 hinterlegt wurde.

**20.** Immunogene Zusammensetzung nach irgendeinem der Ansprüche 1, 2 oder 11 bis 15, **dadurch gekennzeichnet, dass** der Stamm von B. bronchiseptica, ausgehend von welchem das Toxin AC-Hly und die Proteine, die durch die vrg Gene exprimiert werden, erhalten werden, der Stamm 973S ist, der bei der CNCM unter der Nr. I-858 hinterlegt wurde.

**21.** Impfstoffzusammensetzung, **dadurch gekennzeichnet, dass** diese als aktiven Bestandteil eine immunogene Zusammensetzung nach irgendeinem der Ansprüche 1 bis 20 in Verbindung mit einem pharmazeutisch verträglichen Träger und gegebenenfalls einem Hilfsmittel umfasst.

22. Impfstoffzusammensetzung nach Anspruch 21, **dadurch gekennzeichnet, dass** diese als aktiven Bestandteil eine Zusammensetzung nach irgendeinem der Ansprüche 3 bis 6 und eine Zusammensetzung nach irgendeinem der Ansprüche 7 bis 10 umfasst.

23. Impfstoffzusammensetzung nach Anspruch 22, **dadurch gekennzeichnet, dass** diese außerdem eine Zusammensetzung nach irgendeinem der Ansprüche 11 bis 14 umfasst.

24. Impfstoffzusammensetzung nach Anspruch 21, **dadurch gekennzeichnet, dass** diese als aktiven Bestandteil einen "Hamstoffextrakt vag" von B. pertussis und einen "Harnstoffextrakt vrg" von B. pertussis umfasst, wobei der Stamm von B. pertussis vorzugsweise der Stamm HAV ist, der bei der CNCM unter der Nr. I-1485 hinterlegt wurde.

25. Impfstoffzusammensetzung nach Anspruch 21, **dadurch gekennzeichnet, dass** diese als aktiven Bestandteil einen "Hamstoffextrakt vag" von B. parapertussis und einen "Harnstoffextrakt vrg" von B. parapertussis umfasst, wobei der Stamm von B. parapertussis vorzugsweise der Stamm Nr. 1 ist, der bei der CNCM unter der Nr. I-1498 hinterlegt wurde.

26. Impfstoffzusammensetzung nach Anspruch 21, **dadurch gekennzeichnet, dass** diese als aktiven Bestandteil einen "Harnstoffextrakt vag" von B. bronchiseptica und einen "Harnstoffextrakt vrg" von B. bronchiseptica umfasst, wobei der Stamm von B. bronchiseptica vorzugsweise der Stamm 973S ist, der bei der CNCM unter der Nr. I-858 hinterlegt wurde.

27. Impfstoffzusammensetzung nach Anspruch 21, **dadurch gekennzeichnet, dass** diese als aktiven Bestandteil einen "Harnstoffextrakt vag" von B. pertussis und einen "Harnstoffextrakt vrg" von B. pertussis, wobei der Stamm von B. pertus sis vorzugsweise der Stamm HAV ist, der bei der CNCM unter der Nr. I-1485 hinterlegt wurde, und einen "Hamstoffextrakt vag" von B. parapertussis, wobei der Stamm von B. parapertussis vorzugsweise der Stamm Nr. 1 ist, der bei der CNCM unter der Nr. I-1498 hinterlegt wurde, umfasst.

28. Impfstoffzusammensetzung nach Anspruch 27, **dadurch gekennzeichnet, dass** diese außerdem einen "Harnstoffextrakt vag" von B. bronchiseptica umfasst, wobei der Stamm von B. bronchiseptica vorzugsweise der Stamm 973S ist, der bei der CNCM unter der Nr. I-858 hinterlegt wurde.

29. Impfstoffzusammensetzung, **dadurch gekennzeichnet, dass** diese als aktiven Bestandteil eine Mischung von "Harnstoffextrakten vag "und" Harnstoffextrakten vrg" der Stämme von B. pertussis und/oder B. parapertussis und/ oder B. bronchiseptica umfasst.

30. Impfstoffzusammensetzung nach irgendeinem der Ansprüche 21 bis 29, **dadurch gekennzeichnet, dass** diese außerdem als aktiven Bestandteil einen weiteren immunogenen Bestandteil, beispielsweise ein Toxin oder ein inaktives pathogenes Mittel umfasst.

31. Bakterienextrakt, welcher Virulenzfaktoren von Bordetella enthält, die geeignet sind, um in Impfstoffzusammensetzungen eingebracht zu werden, wobei dieser ausgehend von Stämmen der Phase I von B. pertussis, B. parapertussis oder B. bronchiseptica durch die Durchführung der folgenden Schritte erhalten wird:

- Kultivierung von Bakterien auf festem Bordet Gengou-Medium mit Blut (BGS) während 48 Stunden, um isolierte hämolytische Kolonien zu erhalten,

- Resuspendierung einiger Kolonien in flüssigem Stainer-Medium und Ausstreichen von 100 µl dieser Suspension auf festem Stainer-Medium (CSM),

- nach 24 bis 36 Stunden der Kultivierung bei 36°C Resuspendierung der Bakterien in flüssigem Stainer-Medium und Animpfen eines 2 I-Kolbens, welcher 200 ml flüssiges Stainer-Medium enthält, derart, dass die optische Dichte bei 650 nm 0,05 beträgt,

- nach dem Animpfen Kultivierung für 18 bis 20 Stunden bei 36°C unter leichtem Schütteln, um eine optische Dichte bei 650 nm von 1 bis 1,2 zu erhalten,

- Bestimmung der Aktivität von Adenylcyclase in der so erhaltenen Bakteriensuspension ohne Verdünnung. Wenn diese zwischen 30 und 100 U/ml liegt, kann der Hamstoffextrakt hergestellt werden,

- Zentrifugation der Bakteriensuspension für 30 Minuten bei 5000 g bei 4°C,

- Resuspendierung des Bodensatzes der Bakterien in 5 M Harnstoff, hergestellt in dem Puffer PBS, mit Hilfe eines Volumens, das 5 Mal dem Feuchtgewicht des Bodensatzes der Bakterien entspricht,

- Schütteln lassen während 1 Stunde bei 4°C, dann Zentrifugation für 40 Minuten bei 40000 g bei 4°C,

- Konservierung des Überstandes bei -80°C bis zur Verwendung.

32. Bakterienextrakt, welcher Expressionsprodukte der <u>vrg</u>-Gene enthält, die geeignet sind, in Impfstoffzusammensetzungen eingebracht zu werden, wobei dieser ausgehend von Stämmen der Phase IV von <u>B. pertussis</u>, <u>B. parapertussis</u> oder <u>B. bronchiseptica</u> durch die Durchführung der folgenden Schritte erhalten wird:

- Kultivierung von Bakterien auf festem Bordet Gengou-Medium mit Blut (BHS) während 48 Stunden, um isolierte hämolytische Kolonien zu erhalten,

- Resuspendierung einiger Kolonien in flüssigem Stainer-Medium und Ausstreichen von 100 µl dieser Suspension auf festem Stainer-Medium (CSM),

- nach 24 bis 36 Stunden der Kultivierung bei 36°C Resuspendierung der Bakterien in flüssigem Stainer-Medium und Animpfen eines 2 I-Kolbens, welcher 200 ml flüssiges Stainer-Medium enthält, derart, dass die optische Dichte bei 650 nm 0,05 beträgt,

- nach dem Animpfen Kultivierung für 18 bis 20 Stunden bei 36°C unter leichtem Schütteln, um eine optische Dichte bei 650 nm von 1 bis 1,2 zu erhalten,

- Zentrifugation der Bakteriensuspension für 30 Minuten bei 5000 g bei 4°C,

- Resuspendierung des Bodensatzes der Bakterien in 5 M Harnstoff, hergestellt in dem Puffer PBS, mit Hilfe eines Volumens, das 5 Mal dem Feuchtgewicht des Bodensatzes der Bakterien entspricht,

- Schütteln lassen während 1 Stunde bei 4°C, dann Zentrifugation für 40 Minuten bei 40000 g bei 4°C,

- Konservierung des Überstandes bei -80°C bis zur Verwendung.

33. Immunogene Zusammensetzung nach irgendeinem der Ansprüche 1 bis 21, **dadurch gekennzeichnet, dass** der Bakterienextrakt, welcher die Expressionsprodukte der <u>vrg</u>-Gene eines Bordetellastammes, der ausgewählt ist aus <u>B. pertussis</u>, <u>B. parapertussis</u> oder <u>B. bronchiseptica</u>, oder einen Teil dieser Expressionsprodukte enthält, der ausreicht, um bei einem Wirt, welchem der Extrakt verabreicht wird, eine Immunreaktion auszulösen, durch einen Bakterienextrakt eines Stammes von <u>B. pertussis</u>, <u>B. parapertussis</u> oder <u>B. bronchiseptica</u> ersetzt ist, bei welchem das Gen bvgS derart mutiert ist, dass die Bakterien im Wesentlichen die vrg-Gene auf Kosten der <u>vag</u>-Gene exprimieren.

34. Stamm von Bordetella, **dadurch gekennzeichnet, dass** es sich entweder um den Stamm HAV von <u>B. pertussis</u>, der bei der CNCM unter der Nr. I-1485 hinterlegt wurde, oder den Stamm Nr. 1 von <u>B. parapertussis</u>, der bei der CNCM unter der Nr. I-1498 hinterlegt wurde, handelt.

**Claims**

1. Immunogenic composition, **characterized in that** it comprises an adenyl cyclase-hemolysin (AC-Hly) protein, or an immunogenic portion of this AC-Hly, of a strain of Bordetella chosen amongst <u>B. pertussis</u>, <u>B. parapertussis</u> or <u>B. bronchiseptica,</u> and **in that** it comprises in addition, a bacterial extract containing the expression product of the <u>vrg</u> genes of a strain of <u>Bordetella</u> chosen amongst <u>B. pertussis</u>, <u>B. parapertussis</u> or <u>B. bronchiseptica,</u> or a portion of these expression products which is sufficient to induce an immune response in a host to which the extract might be administered.

2.  Immunogenic composition according to claim 1, **characterized in that** it comprises, in addition, one or more adhesins or toxins of B. pertussis, B. parapertussis or B. bronchiseptica, chosen amongst FHA, the AGGs or PRN and PTX.

3.  Immunogenic composition according to claim 1 or claim 2, **characterized in that** it comprises the PTX toxin of B. pertussis, the AC-Hly toxin of B. pertussis and a bacterial extract containing the proteins encoded by the vrg genes of B. pertussis, or a portion of these proteins which is sufficient to induce an immune response in a host to which the extract might be administered.

4.  Immunogenic composition according to claim 3, **characterized in that** the AC-Hly toxin of B. pertussis is contained in a bacterial extract containing all or part of the virulence factors of the adhesins or toxins groups of B. pertussis.

5.  Immunogenic composition according to claim 4, **characterized in that** the AC-Hly toxin of B. pertussis is present in the form of a urea extract termed "vag urea extract".

6.  Immunogenic composition according to any one of claims 1 to 4, **characterized in that** the AC-Hly toxin of B. pertussis is present in the form of a urea extract termed "vag urea extract", and **in that** the bacterial extract containing all or part of the proteins encoded by the vrg genes of B. pertussis is also a urea extract termed "vrg urea extract".

7.  Immunogenic composition according to claim 1 or claim 2, **characterized in that** it comprises the AC-Hly toxin of B. parapertussis and a bacterial extract containing all or part of the proteins encoded by the vrg genes of B. parapertussis.

8.  Immunogenic composition according to claim 7, **characterized in that** the AC-Hly toxin of B. parapertussis is contained in a bacterial extract containing all or part of the virulence factors of the adhesins or toxins groups of B. parapertussis.

9.  Immunogenic composition according to claim 8, **characterized in that** the AC-Hly toxin of B. parapertussis is present in the form of a urea extract termed "vag urea extract".

10.  Immunogenic composition according to any one of claims 1, 2, 7 or 8, **characterized in that** the AC-Hly toxin of B. parapertussis is present in the form of a urea extract termed "vag urea extract", and **in that** the bacterial extract containing all or part of the proteins encoded by the vrg genes of B. parapertussis is also a urea extract termed "vrg urea extract".

11.  Immunogenic composition according to claim 1 or claim 2, **characterized in that** it comprises the AC-Hly toxin of B. bronchiseptica and a bacterial extract containing all or part of the proteins encoded by the vrg genes of B. bronchiseptica.

12.  Immunogenic composition according to claim 11, **characterized in that** the AC-Hly toxin of B. bronchiseptica is contained in a bacterial extract containing all or part of the virulence factors of the adhesins or toxins groups of B. bronchiseptica.

13.  Immunogenic composition according to claim 12, **characterized in that** the AC-Hly toxin of B. bronchiseptica is present in the form of a urea extract termed "vag urea extract".

14.  Immunogenic composition according to any one of claims 1, 2, 11 or 12, **characterized in that** the AC-Hly toxin of B. bronchiseptica is present in the form of a urea extract termed "vag urea extract", and **in that** the bacterial extract containing all or part of the proteins encoded by the vrg genes of B. bronchiseptica is also a urea extract termed "vrg urea extract".

15.  Immunogenic composition according to any one of claims 1, 2, or 11 to 14, **characterized in that** it comprises, in addition, polypeptide components characteristic of the flagella of B. bronchiseptica.

16.  Immunogenic composition according to claim 1 or claim 2, **characterized in that** the AC-Hly toxin is in pure form.

**17.** Immunogenic composition according to claim 16, **characterized in that** the toxin is replaced by AC-Hly fragments containing at least the C-terminal portion of the toxin and/or at least one internal domain of AC-Hly.

**18.** Immunogenic composition according to any one of claims 1 to 6, **characterized in that** the B. pertussis strain from which the AC-Hly toxin and the proteins expressed by the vrg genes are obtained, is the strain HAV deposited at the CNCM under No. I-1485.

**19.** Immunogenic composition according to any one of claims 1, 2, 7 to 10, **characterized in that** the B. parapertussis strain from which the AC-Hly toxin and the proteins expressed by the vrg genes are obtained is the strain No. 1 deposited at the CNCM under No. I-1498.

**20.** Immunogenic composition according to any one of claims 1, 2, or 11 to 15, **characterized in that** the B. bronchiseptica strain from which the AC-Hly toxin and the proteins expressed by the vrg genes are obtained is the strain 973S deposited at the CNCM under No. I-858.

**21.** Vaccinating composition, **characterized in that** it comprises as active principle an immunogenic composition according to any one of claims 1 to 20, in combination with a pharmaceutically acceptable vehicle and, where appropriate, an adjuvant.

**22.** Vaccinating composition according to claim 21, **characterized in that** it comprises, as active principle, a composition according to any one of claims 3 to 6 and a composition according to any one of claims 7 to 10.

**23.** Vaccinating composition according to claim 22, **characterized in that** it comprises in addition, a composition according to any one of claims 11 to 14.

**24.** Vaccinating composition according to claim 21, **characterized in that** it comprises as active principle a "vag urea extract" of B. pertussis and a "vrg urea extract" of B. pertussis, the B. pertussis strain preferably being strain HAV deposited at the CNCM under No. I-1485.

**25.** Vaccinating composition according to claim 21, **characterized in that** it comprises as active principle a "vag urea extract" of B. parapertussis and a "vrg urea extract" of B. parapertussis, the B. parapertussis strain preferably being strain No. 1 deposited at the CNCM under No. I-1498.

**26.** Vaccinating composition according to claim 21, **characterized in that** it comprises as active principle a "vag urea extract" of B. bronchiseptica and a "vrg urea extract" of B. bronchiseptica, the B. bronchiseptica strain preferably being the strain 973S deposited at the CNCM under No. I-858.

**27.** Vaccinating composition according to claim 21, **characterized in that** it comprises as active principle a "vag urea extract" of B. pertussis and a "vrg urea extract" of B. pertussis, the B. pertussis strain preferably being the strain HAV deposited at the CNCM under No. I-1485, and a "vag urea extract" of B. parapertussis, the B. parapertussis strain preferably being the strain No. 1 deposited at the CNCM under No. I-1498.

**28.** Vaccinating composition according to claim 27, **characterized in that** it comprises, in addition, a "vag urea extract" of B. bronchiseptica, the B. bronchiseptica strain preferably being the strain 973S deposited at the CNCM under No. I-858.

**29.** Vaccinating composition, **characterized in that** it comprises as active principle a mixture of "vag urea extracts" and "vrg urea extracts" from B. pertussis and/or B. parapertussis and/or B. bronchiseptica strains.

**30.** Vaccinating composition according to any one of claims 21 to 29, **characterized in that** it contains, in addition, as active principle, another immunogenic constituent, for example an inactivated pathogenic agent or toxin.

**31.** Bacterial extract containing virulence factors of Bordetella, which is capable of participating in vaccinating compositions, as is obtained from phase I strains of B. pertussis, B. parapertussis or B. bronchiseptica by carrying out the following steps:

- culture of the bacteria on solid blood Bordet-Gengou medium (BBG) for 48 hours so as to obtain isolated haemolytic colonies,

- resuspension of a few colonies in liquid Stainer medium and plating out of 100 μl of this suspension on solid Stainer medium (CSM),
- after 24-36 hours of culture at 36°C, resuspension of the bacteria in liquid Stainer medium and inoculation of a 2-1 flask containing 200 ml of liquid Stainer medium such that the optical density at 650 nm is 0.05,
- after inoculation, culture for 18-20 hours at 36°C with gentle agitation so as to obtain an optical density at 650 nm of 1-1.2,
- assay of the adenyl cyclase activity of the bacterial suspension thereby obtained without dilution. If this activity is between 30 and 100 U/ml, the urea extract may be prepared,
- centrifugation of the bacterial suspension for 30 minutes at 5000 g at 4°C,
- resuspension of the bacterial pellet in 5M urea prepared in PBS buffer in the proportion of a volume equal to 5 times the wet weight of the bacterial pellet,
- leaving stirring for 1 hour at 4°C, then centrifugation for 40 minutes at 40000 g at 4°C,
- storage of the supernatant at -80°C until use.

32. Bacterial extract containing expression products of the vrg genes, which is capable of participating in vaccinating compositions, as is obtained from phase IV strains of B. pertussis, B. parapertussis or B. bronchiseptica by carrying out the following steps:

- culture of the bacteria on solid blood Bordet-Gengou medium (BBG) for 48 hours so as to obtain isolated haemolytic colonies,
- resuspension of a few colonies in liquid Stainer medium and plating out of 100 μl of this suspension on solid Stainer medium (CSM),
- after 24-36 hours of culture at 36°C, resuspension of the bacteria in liquid Stainer medium and inoculation of a 2-1 flask containing 200 ml of liquid Stainer medium such that the optical density at 650 nm is 0.05,
- after inoculation, culture for 18-20 hours at 36°C with gentle agitation so as to obtain an optical density at 650 nm of 1-1.2,
- centrifugation of the bacterial suspension for 30 minutes at 5000 g at 4°C,
- resuspension of the bacterial pellet in 5M urea prepared in PBS buffer in the proportion of a volume equal to 5 times the wet weight of the bacterial pellet,
- leaving stirring for 1 hour at 4°C, then centrifugation for 40 minutes at 40000 g at 4°C,
- storage of the supernatant at -80°C until use.

33. Immunogenic composition according to any one of claims 1 to 21, **characterized in that** the bacterial extract containing the expression products of the vrg genes of a strain of Bordetella chosen amongst B. pertussis, B. parapertussis or B. bronchiseptica, or a portion of these expression products which is sufficient to induce an immune response in a host to which the extract might be administered, is replaced, by the bacterial extract of a B. pertussis, B. parapertussis or B. bronchiseptica strain whose bvgS gene is mutated in such a way that the bacterium essentially expresses the vrg genes to the detriment of the vag genes.

34. Bordetella strain, **characterized in that** it is either the B. pertussis strain HAV deposited at the CNCM under No. I-1485, or the B. parapertussis strain No. 1 deposited at the CNCM under No. I-1498.

Détection d'anticorps sériques après
Immunisation par un extrait urée vag
*Bordetella bronchiseptica*, inactivé ou non gel de polyacrylamide 8-25%

antigène: extrait urée *Bordetella bronchiseptica* après passage sur colonne séphadex G25

sérum A: immunsérum de souris immunisées avec 2x25 µg d'extrait urée vag *Bordetella bronchiseptica* non inactivé

sérum B: immunsérum de souris immunisées avec 2x25 µg d'extrait urée vag *Bordetella bronchiseptica* inactivé à la glutaraldéhyde

**Figure 1**

LYMPHOPROLIFERATION:
VACCIN EXTRAIT UREE vag
BORDETELLA BRONCHISEPTICA

Figure 2

**INFECTION RESPIRATOIRE
A BORDETELLA BRONCHISEPTICA**

Témoins
Vaccin Bb973
EU vag Bb973
EUi vag Bb973

**log CFU/poumon**

**nombre de jours après l'infection**

**Figure 3**

EP 0 719 560 B1

**FIGURE 4A**

B+: 9.73 vag
B-: 9.73 vrg
AC: AC-Hly

FIGURE 4B

**FIGURE 4C**

FIGURE 4D

**INFECTION RESPIRATOIRE
A BORDETELLA BRONCHISEPTICA
Figure 5**

nombre de jours après l'infection